# NOUVEAU FASCICULE DE BREVET EUROPEEN

(11) **EP 0 549 776 B2**
(45) Date de publication et mention de la décision concernant l'opposition: **27.03.2002**
(45) Mention de la délivrance du brevet: 02.12.1998
(21) Numéro de dépôt: 92916146.1
(22) Date de dépôt: 17.07.1992
(51) Int. Cl.: C12Q 1/68, C07H 21/00

(54) **SYSTEME DE SONDES PERMETTANT D'EFFECTUER LE TYPAGE HLA DR, ET PROCEDE DE TYPAGE UTILISANT LESDITES SONDES**
Sonden System zur HLA-DR Charakterisierung und sie verwendendes Charakterisierungs-Verfahren
PROBE SYSTEM FOR PERFORMING HLA DR TYPING AND TYPING METHOD USING SAID PROBES

(30) Priorité: 17.07.1991 FR 9109058
(43) Date de publication de la demande: 07.07.1993
(73) Titulaire: BIO MERIEUX, Société anonyme, F-69280 Marcy l'Etoile (FR)
(72) Inventeur: ALLIBERT, Patrice, André, F-69290 Grézieu-la-Varenne (FR); GROS, Philippe, F-69005 Lyon (FR); MACH, Bernard, François, CH-1292 Chambesy (CH); MANDRAND, Bernard, Fabien, F-69100 Villeurbanne (FR); TIERCY, Jean-Marie, CH-1224 Chêne-Bougeries (CH)
(74) Mandataire: Tonnellier, Jean-Claude
(86) Numéro de dépôt international: FR9200702
(87) Numéro de publication internationale: WO9302213

(56) Documents cités:
- EP-A- 0 237 362
- EP-A- 0 433 748
- EP-A- 0 456 304
- EP-A- 0 472 399
- EP-B- 0 466 367
- WO-A-89/04875
- WO-A-89/11547
- WO-A-92/10589
- WO-A-93/09245
- JP-A- 3 164 180
- US-A- 4 965 189
- PROCEEDINGS OF THE NATIONAL ACADEMY OF SCIENCES OF USA. vol. 85, 15 Mai 1988, WASHINGTON US pages 3504 - 3508 S. J. SCHARF ET AL. 'HLA class II allelic variation and susceptibility to pemphigus vulgaris'
- HUMAN IMMUNOLOGY vol. 30, no. 4, 1991, NEW YORK US pages 278 - 284 B. MACH ET AL. 'Gnotypic typing of HLA class II: from the bench to the bedside' cité dans la demande
- Vaughan R W et al., 1990, Tissue Antigens, vol.36, pp 149-155
- Ehrlich H et al., Février-Avril, European Journal of Immunogenetics, vol.18, pp 33-55
- Scharf S J et al., Mars 1991, Human Immunology, vol. 30, pp 190-201
- Nevinny-Stickel C et al., Octobre-Décembre 1991, European Journal of Immunogenetics, vol. 18 pp 323-332
- Martell R W et al., Mai 1991, Tissue Antigens, vol. 37, pp 232-234
- Martin F H et al., 1985 Nucleic Acids Research, vol. 13, pp 8927- 8938

## Description

La présente invention a pour objet un procédé pour déterminer le génotype HLA de classe II d'un individu et se rapporte plus particulièrement à la détection des gènes polymorphes HLA DR. Ce procédé est applicable notamment au typage HLA en transplantation, au diagnostic médical, à la médecine légale, etc.

Le système HLA (antigène des lymphocytes humains) est codé par le complexe d'histocompatibilité majeur chez l'homme. Il constitue une contrainte très importante au cours des transplantations d'organes entre les individus en effectuant une distinction entre le soi et le non-soi. De plus, les facteurs du HLA sont impliqués dans la prédisposition à un grand nombre de maladies. Les antigènes du système HLA ont donc été utilisés dans des procédés de typage pour déterminer les caractéristiques entre donneurs et receveurs lors de transplantations d'organes (F. H. BACH et J. J. VAN ROOD, N. Engl. J. Med., 295, pages 806-13 (1976)), ainsi que la prédisposition d'un individu à certaines maladies.

D'un point de vue génétique, le système H LA est bien caractérisé et consiste en un ensemble de loci plus ou moins polymorphes situés dans un intervalle d'environ 2 centimorgans (cM) sur le bras court du chromosome 6. Trois loci dans ce système (HLA-A, B et C) codent pour une classe d'alloantigènes exprimés de façon codominante (classe I). Une autre région (HLA-D) qui contient en fait plusieurs gènes code pour une seconde classe d'alloantigènes exprimés de façon codominante avec un degré important de polymorphisme (classe II). Plusieurs autres loci qui contrôlent notamment les composants C2, C4 et le facteur Bf de la cascade du complément appartiennent également au système HLA (classe III). Le succès des greffes d'organes dépend dans une grande mesure de l'identité HLA (classes I et II) entre receveur et donneur. En conséquence, le typage HLA doit être le plus exact possible. Ce besoin concerne principalement les transplantations de reins (P.J. Morris et A. Ting (1982) Immunol. Rev 66, 103 - G. Opelz (1989) Transpl. Proc. 21,609 - E.L. LAGAAIJ, P.H. Hennemann, M. Ruigrok et al. (1985) New Engl. J. Med 321,701) et les greffes de moelle osseuse (P.G. Beatty, R.A. Clift, E.M. Mickelson et al. (1985) New Engl. J. Med 313,765 - J.M. Hows et B.A. Bradley (1990) British J. Hematol. 76,1). Dans le cadre de la greffe de moelle osseuse, l'identité parfaite au niveau des antigènes HLA classe II représente un facteur déterminant pour le succès de la greffe, c'est-à-dire pour éviter un rejet du greffon ou le développement d'une maladie greffon-contre-hôte (P.G. Beatty, J. Hansen, G.M. Longton et al. (1991) Transplantation 51, 443 - R.C. Ash, J.T. Casper, C.R. Chitambar et al. (1990) New Engl. J. Med. 322, 485 - C. Anasetti, D. Amos, P.G. Beatty et al. (1989) New Engl. J. Med. 320,197).

Le polymorphisme des produits d'expression des gènes de la région HLA D est défini habituellement par des techniques sérologiques basées sur l'analyse par des alloantisera des produits des gènes HLA exprimés à la surface des cellules (J.J. Van Rood et A. Van Leeuwen (1963) J. Clin. Invest. 42,1382 - J.J. Van Rood, A. Van Leeuwen, J.J. Koning, A.B. Van Oud Ablas (1975) Tissue Antigens 5, 73). La précision et la reproductibilité dépendent des lots de sera disponibles. Cependant, même dans les meilleures conditions, un très grand nombre des allèles existants ne sont pas détectables par ces techniques sérologiques. Les limites de l'analyse sérologique résultent essentiellement de l'absence d'alloantiséra mono-spécifiques, d'une discrimination incomplète avec des réactivités croisées entre des spécificités très proches, par exemple DR3 et DRw13 ou encore d'une expression altérée des molécules HLA classe Il à la surface des cellules, par exemple de cellules leucémiques.

Grâce à la biologie moléculaire, on sait maintenant qu'il existe un plus grand nombre de gènes HLA qu'on ne le supposait auparavant et, surtout, beaucoup plus d'allèles différents. Cette diversité est maintenant caractérisée au niveau des séquences d'ADN des différents gènes et allèles. Selon le dernier rapport du comité de nomenclature HLA (voir The WHO Nomenclature Committee for factors of the HLA system (1990) Immunogenetics 31, 131- and - J.G. Bodmer, S.G.E. Marsh, E.D. Albert, W.F. Bodmer, B. Dupont, H.A. Erlich, B. Mach, W.R. Mayr, P. Parham, T. Sasazuki, G.M.T. Schreuder, J.L. Strominger, A. Svejgaard et P.I. Terasaki (1991) Tissue Antigens 37, 97), le polymorphisme HLA classe Il se répartit comme suit : locus DRB1 : 47 allèles, locus DRB3 : 4 allèles, locus DRB4 : 1 allèle, locus DRB5 : 4 allèles, locus DQB1 : 17 allèles, locus DQA1 : 13 allèles, locus DPB1 : 21 allèles, locus DPA1 : 4 allèles.

Beaucoup de ces allèles échappent à l'analyse sérologique et ne sont identifiables qu'au niveau de l'ADN. On peut illustrer les limites du typage sérologique par la spécificité sérologique DR4, maintenant subdivisée en 11 sous-types (DRB1*0401-0411) (voir J.G. Bodmer, S.G.E. Marsh, E.D. Albert, W.F. Bodmer, B. Dupont, H.A. Erlich, B. Mach, W.R. Mayr, P. Parham, T. Sasazuki, G.M.T. Schreuder, J.L. Strominger, A. Svejgaard et P.I. Terasaki (1991) Tissue Antigens 37, 97), identifiables seulement au niveau de la séquence d'ADN.

De même, la spécificité DRw6, qui peut être subdivisée en DRw13 et DRw14 par quelques alloantisera, contient en réalité 10 séquences alléliques (DAB1*1301-1305 et DAB1*1401-1405 (voir la publication de Bodmer JG citée ci-dessus) qui, là aussi, ne peuvent être discriminées que par l'analyse génotypique au niveau de la séquence d'ADN.

L'analyse génotypique est une nouvelle approche permettant d'analyser la diversité du système HLA classe II directement au niveau des gènes. L'analyse génotypique est basée sur le principe de l'hybridation moléculaire et la première approche qui fut proposée est la technique dite "RFLP" qui consiste à fragmenter l'ADN par utilisation d'enzymes de restriction et à analyser la taille des fragments d'ADN spécifiques générés par ces enzymes (voir C.T. Wake, E.O. Long et B. Mach (1982) Nature 300, 372 - J. Bîhme, M; Andersson, G. Andersson, E. Miller, P.A. Peterson et L. Rask (1985) J. Immunol. 135, 2149 - J.L. Bidwell, E.A. Bidwell, D.A. Savage, D. Middleton, P.T. Klouda et B.A. Bradley (1988) Transplantation 45, 640).

L'analyse par RFLP ne permet de reconnaître que certaines des différences alléliques indétectables par la sérologie, et cette technique présente encore des limitations. En effet, un allèle portant une séquence différente est identifiable seulement si le nucléotide différent est dans le site de reconnaissance de l'enzyme de restriction utilisée dans l'analyse et donc un grand nombre d'allèles HLA classe II ne seront pas reconnus par cette analyse. De plus, l'analyse RFLP met rarement en évidence une modification dans une séquence codante, et ne fournit pas d'informations sur la nature exacte de la modification. Enfin, cette technique est longue et lourde car elle implique d'utiliser des quantités relativement importantes d'acides nucléiques qui doivent être digérées avec plusieurs enzymes de restriction, des électrophorèses et des transferts sur filtres.

Pour illustrer les limites de la technique RFLP, on peut mentionner que les sous-types des spécificités DR1, DR4, DRw8, DRw11 ou DRw13, ne sont pas détectables par RFLP.

Une nouvelle technique d'analyse génotypique de HLA classe II a été proposée, qui est la méthode dite "de typage par oligonucléotides". Grâce à la connaissance des séquences d'ADN des gènes de HLA classe II et en particulier des gènes DRβ, qui sont de loin les plus polymorphes, on peut utiliser des oligonucléotides qui sont spécifiques d'un endroit donné de la séquence du gène comme traceurs pour l'analyse du polymorphisme par hybridation. Ces oligonucléotides sont choisis de façon à être les plus informatifs possibles et à permettre l'identification des différents allèles, sur la base de leurs différences de séquences. Dans la pratique, n'importe quelle différence de séquence, même d'un seul nucléotide, peut être détectée.

La technique de typage par oligonucléotides peut être appliquée aussi bien à l'ADN, comme décrit dans la publication d'Angelini et al., Proc. Natl. Acad. Sci. USA Vol. 83, pages 4489 - 4493 (1986), qu'à l'ARN (voir C. Ucla, J.J. Van Rood, J. Gorski et B. Mach (1987) J. Clin. Invest. 80, 1155).

Cette nouvelle approche est basée sur le principe de l'hybridation moléculaire en utilisant les propriétés caractéristiques des acides nucléiques qui sont les possibilités d'interagir avec une séquence complémentaire par l'intermédiaire de liaisons hydrogènes et de former ainsi un hybride stable, selon les lois d'appariement connues, c'est-à-dire A-T, G-C, pour l'ADN et AU, G-C pour l'ARN. Ainsi, des oligonucléotides de synthèse correspondant à des séquences d'ADN ou d'ARN des allèles connus peuvent être utilisés comme sondes pour identifier, dans un échantillon, une séquence nucléique appelée cible, contenant une séquence complémentaire de celle de la sonde. Le marquage de l'hybride formé entre la cible et la sonde permet la détection et la quantification de la cible dans l'échantillon. Ce marquage est effectué par tout marqueur connu, tel que marqueur enzymatique, chimique ou radioactif. Sur la base de ces principes la première application de typage par oligonucléotide pour le HLA classe II a été présentée par Angelini et al. dans la publication précédemment citée, avec utilisation de la technique dite "SOUTHERN" selon laquelle l'ADN cible est déposé sur une membrane de nylon et la détection est effectuée à l'aide d'une sonde oligonucléoditique marquée. La technique a ensuite été appliquée à la détection d'allèles HLA classe Il non identifiables par la sérologie de routine (voir J.M. Tiercy, J. Gorski, M. Jeannet et B. Mach (1988) Proc. Natl. Acad. Sci. USA 85, 198 - J.M. Tiercy, J. Gorski, H. Bétuel, A.C. Freidel, L. GebÅhrer, M. Jeannet et B. Mach (1989) Human Immunol. 24, 1). Une autre application directe au typage HLA classe II est celle décrite dans la demande de brevet PCT WO 89/11547 utilisant la technique dite "Dot-Blot". Une modification à ces techniques est représentée par la méthode dite "Reverse Dot Blot" qui consiste à fixer sur une membrane de papier, nitrocellulose ou un mélange des deux, une sonde nucléotidique et à effectuer la détection d'une hybridation avec une cible marquée. Cette technique a été appliquée au typage HLA-DQA et à la détection des mutations de la β-thalassémie méditerranéenne (R.K. Saiki et al., Proc. Natl. Acad. Sci. USA, Vol 86, pages 6230-6234 (1989)).

Comme décrit précédemment et expliqué dans les publications et la demande de brevet citées ci-dessus, le typage cellulaire nécessite la détection de mutations ponctuelles dans le génome et implique la mise au point de sondes suffisamment sensibles pour détecter et différencier des séquences homologues à un nucléotide près, et on a trouvé qu'il fallait utiliser des sondes de courte taille, généralement de moins de 30 nucléotides, qui confèrent au test une grande spécificité, tout en conservant une bonne sensibilité. L'utilisation d'oligonucléotides de courte taille permet de disposer d'un grand éventail de sélectivité.

Dans le cas où l'on utilise un test comprenant la fixation d'une sonde sur un support solide, reste posé le problème lié à l'immobilisation d'une sonde courte, inférieure à 30 nucléotides, sur un tel support solide. R.K. SAIKI et al., dans la publication précédemment citée, ont proposé une méthode qui consiste à coupler à l'extrémité 3' d'une sonde comprenant entre 15 et 20 bases, une queue poly(dT) de 400 bases et à immobiliser la sonde par l'intermédiaire de cette queue sur un filtre de nylon par exposition à des rayons ultraviolets, de façon à coupler par covalence les bases thymines aux amines primaires présentes dans le nylon.

Cependant, cette méthode n'est pas entièrement satisfaisante, car elle présente des problèmes de spécificité. En effet, les bases thymines de la sonde peuvent également réagir, sous rayonnement U.V., avec le support, ce qui implique une diminution de l'efficacité d'hybridation.

Par ailleurs, pour des raisons d'industrialisation, il est souhaitable de mettre au point un procédé de typage qui présente une grande spécificité et une bonne sensibilité, mais qui de plus soit simple à mettre en oeuvre, d'exécution rapide, peu onéreux, automatisable et utilisable pour le typage individuel.

On a maintenant trouvé un nouveau procédé pour déterminer le génotype HLA DR d'un individu, qui pallie les inconvénients décrits ci-dessus en permettant de détecter et de différencier des séquences homologues à un nucléotide près.

Le procédé de l'invention est mis en oeuvre à l'aide d'un ensemble de sondes nucléotidiques choisies de façon à permettre un typage avec un nombre minimum de sondes. Cet ensemble de sondes présente notamment l'avantage de permettre d'opérer à une température unique, notamment à 37° C. (bien qu'il soit possible d'opérer à une autre température, comme on le verra dans la partie expérimentale ci-après). Un tel ensemble de sondes fait également partie de l'invention.

Le document JP-3-164 180 décrit un ensemble de sondes nucléotidiques dont l'une présente une homologie partielle avec l'une des sondes de l'ensemble de sondes décrit dans la présente demande.

L'ensemble des sondes de l'invention qui sera défini ci-après, peut être utilisé sous la forme de sondes de détection (marquées avec un agent traceur usuel) dans les techniques du type Southern, ou, de préférence, sous la forme de sondes de capture (technique sandwich ou reverse dot blot) immobilisées sur un support solide, soit par fixation passive (adsorption) directement ou par l'intermédiaire d'un ligand), tel qu'un ligand hydrophobe (voir par exemple la demande de brevet européen N° 0 405 913), soit par l'établissement d'au moins une liaison covalente qui peut être faite ici encore directement ou par l'intermédiaire d'un ligand capable de se fixer par covalence sur le support (voir par exemple la demande de brevet PCT N° WO 88/01302). L'immobilisation des sondes peut être réalisée soit à l'aide de procédés connus, soit à l'aide d'autres procédés qui seront décrits ci-après.

Il est évident pour les spécialistes qu'à chaque sonde nucléotidique particulière correspond une sonde complémentaire, qui est bien entendu capable de jouer le même rôle en tant que sonde de capture ou de détection. L'invention s'étend donc à de telles sondes ayant une séquence complémentaire de celles qui seront décrites ci-après.

L'invention a donc pour objet un ensemble de sondes permettant de déterminer selon les techniques de typage par oligonucléotides, les types HLA DRβ et/ou les sous-types associés, un procédé pour déterminer le typage HLA DR, et des sondes individuelles permettant la mise en oeuvre de ce procédé. Cet ensemble de sondes, ce procédé et ces sondes individuelles sont tels que définis dans les revendications annexées. La sonde 34bis peut être notamment utilisée sous la forme :

AACCAGIAGGAGAACGT

L'invention conceme notamment un ensemble de sondes tel que défini ci-dessus, caractérisé par le fait qu'il comprend en outre les sondes suivantes (la partie soulignée correspondant à la séquence optimum) : séquences 42 et 42bis, et/ou les suivantes : 52, 37, 55.

(G)AGGAGGACTTGCGC(T) - (T)ACGGGGCTGTGGA(G)
- (G)GAGCTGCGTAAG(T) - TTCCTGGAGAGACAC - (G)GGAGAGATACTTC(C)

L'invention a également pour objet un procédé pour déterminer le typage HLA DRβ d'un individu, selon les techniques usuelles de typage par oligonucléotides, caractérisé par le fait que l'on utilise comme sondes de capture ou de détection, soit séquentiellement, soit simultanément, au moins une partie des sondes de l'ensemble de sondes tel que défini ci-dessus :

Dans un procédé automatisé, on utilisera l'ensemble des sondes. Dans d'autres cas, il est évidemment possible de les utiliser l'une après l'autre, et d'arrêter le procédé lorsque les renseignements recueillis suffisent à déterminer le typage.

Le procédé de l'invention comprend donc essentiellement les étapes consistant :
- à mettre en contact selon une technique particulière choisie, des échantillons d'acides nucléiques cibles contenant les régions polymorphes du gène HLA DR d'un individu, avec au moins une partie de l'ensemble des sondes tel que défini ci-dessus,
- à faire incuber, selon les méthodes connues, dans des conditions prédéterminées telles que l'hybridation avec chaque sonde ne se produit que si la cible contient une séquence parfaitement complémentaire de celle de ladite sonde, et
- à déterminer, selon les techniques de détection usuelles, l'hybridation ou l'absence d'hybridation avec chacune des sondes utilisées.

Les informations recueillies sont ensuite utilisées pour déterminer le typage en fonction d'un plan de typage préétabli, tenant compte des sondes utilisées et de la connaissance des types HLA DR et/ou sous types associés répertoriés. Ce travail est simplifié par l'utilisation d'un plan de typage, c'est à dire en pratique un tableau donnant directement les types et/ou sous types en fonction des réponses positives (hybridation(s)) observées. Pour l'ensemble des sondes de la présente invention, un tel tableau est donné ci-après dans la partie expérimentale (voir tableau 6).

L'invention concerne en particulier un procédé tel que défini ci-dessus, dans lequel on utilise lesdites sondes comme sondes de capture, ce procédé pouvant être caractérisé par le fait qu'il comprend les étapes consistant à:
a) immobiliser chaque sonde de capture sur un support solide,
b) mettre en contact chaque sonde de capture immobilisée avec un milieu liquide contenant au moins un fragment d'acide nucléique cible, dans des conditions prédéterminées permettant l'hybridation si la séquence complémentaire de celle de la sonde est présente dans la cible et
c) détecter la présence d'hybrides éventuellement formés.

Bien entendu, les sondes de l'invention permettent de détecter aussi bien des fragments cibles d'ARN que d'ADN. Par ailleurs,il est évident que l'on peut utiliser comme sonde de détection outre les sondes ci-dessus,toutes sondes appropriées, notamment l'une des sondes décrites ci-après dans l'exemple 5.

Lorsque la sonde de capture est très courte, c'est-à-dire inférieure à 20 nucléotides et en particulier inférieure à 17 nucléotides, il devient nécessaire de mettre en oeuvre des moyens permettant d'améliorer la fixation de la sonde sur un support solide. La fixation de la sonde sur le support est effectuée alors sous la forme d'un dérivé résultant du couplage covalent de la sonde avec un ligand facilitant la fixation sur le support solide. Le ligand, qui peut comprendre une partie hydrophobe est notamment un ligand comprenant au moins un groupement fonctionnel polaire, par exemple un groupement aminé. Le groupement fonctionnel peut servir à fixer la sonde sur le suppport solide par établissement d'une liaison covalente. Lorsque le groupement fonctionnel polaire ne réagit pas avec le support, il améliore la fixation par adsorption sur le support, même si le support est hydrophobe.

Le ligand est par exemple choisi parmi les protéines et les composés tels que représentés respectivement par les formules I et II ci-dessous : 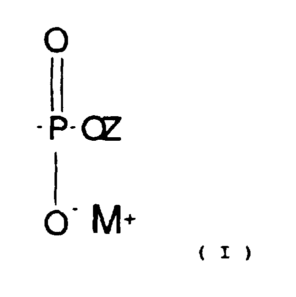 dans laquelle :
Z représente un radical alkyle ou alcényle de 2 à 12 atomes de carbone, linéaire ou ramifié, non substitué ou substitué par un ou plusieurs groupements choisis parmi les groupements hydroxy et/ou amino, et m+ représente notamment un ion alcalin ou ammonium.

Ce ligand est couplé préférentiellement à l'extrémité 5' de la séquence nucléotidique de la sonde de capture 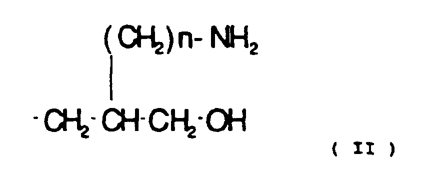 dans laquelle n est un nombre entier pouvant varier de 1 à 4 et de préférence n = 1 ou 4.

Ce ligand est couplé préférentiellement à l'extrémité 3' de la séquence nucléotidique de la sonde de capture.

Lorsque le ligand est une protéine, on choisit par exemple une albumine, de préférence l'albumine de sérum de bovin, qui peut être couplée à l'extrémité 5' ou 3' de la séquence nucléotidique de la sonde de capture.

Le support de la présente invention peut être tout support permettant d'immobiliser une séquence nucléotidique ou un dérivé selon l'invention, soit par adsorption passive soit par covalence. Les supports peuvent être réalisés en tout matériau habituellement utilisé tel que nitrocellulose, nylon, papier, ou de préférence, en un matériau hydrophobe tel qu'un polymère de styrène ou un copolymère à base de styrène comprenant au moins 10 % en poids de motifs styrène.

Le support solide selon l'invention peut être sans limitation sous la forme d'une plaque de microtitration, d'une feuille, d'un tube, d'un cône, de puits ou analogues.

Selon le procédé de la présente invention, un échantillon contenant un acide nucléique est obtenu à partir d'un individu dont le génotype HLA DR doit être déterminé. Tout type de tissu contenant de l'acide nucléique HLA DR peut être utilisé dans le cadre de la présente invention. Il est ainsi possible d'utiliser des fragments d'acide nucléique (ADN ou ARN) obtenus après coupure par voie chimique, enzymatique ou analogue de l'acide nucléique présent dans l'échantillon de l'individu.

Cependant l'incorporation d'une étape préalable d'amplification de l'ADN ou de l'ARN cible peut faciliter le procédé de typage par oligonucléotide de la présente invention. Le principe de l'analyse du polymorphisme HLA par hybridation d'oligonucléotides spécifiques de séquences reste le même, mais une étape d'amplification sélective permet un enrichissement en séquences de la cible, ce qui simplifie la technique (R.K. Saiki, T.L. Bugawan, G.T. Hom, K.B. Mullis et H.A. Erlich (1986) Nature 324, 163 - J.M. Tiercy, M. Jeannet et B. Mach (1990) Eur. J.Immunol. 20, 237).

L'amplification peut être obtenue soit à partir d'ADN, soit à partir d'ARN. Il est évident pour un homme du métier que l'amplification des séquences de la cible HLA DR dans un échantillon peut être accomplie par toute méthode connue qui permet d'obtenir une amplification suffisante pour que la séquence de la cible puisse être détectée par hybridation d'un acide nucléique à une sonde.

En général, l'acide nucléique dans l'échantillon sera de l'ADN, le plus souvent de l'ADN génomique. Cependant, la présente invention peut également être mise en oeuvre avec d'autres acides nucléiques tels que de l'ARN messager ou de l'ADN cloné, et l'acide nucléique dans l'échantillon de l'individu pourra être sous la forme d'un simple brin ou d'un double brin. Bien entendu, lorsque l'acide nucléique est sous la forme double brin, il est nécessaire de pratiquer une étape de dénaturation pour obtenir un acide nucléique simple brin.

Les sondes utilisées dans la présente invention sont des oligonucléotides spécifiques d'une séquence (OSS) qui, dans des conditions appropriées, peuvent se lier spécifiquement à leurs séquences complémentaires. Si une sonde particulière peut être utilisée pour identifier uniquement un allèle, la sonde est alors appelée OSA, c'est-à-dire oligonucléotide spécifique d'un allèle. Il est possible qu'une seule sonde ne soit pas capable d'identifier à elle seule un allèle spécifique DRβ en raison de la nature différente entre divers allèles DRβ.

Selon le procédé de l'invention, l'identité d'un allèle est déduite à partir d'un modèle de liaison d'un ensemble de sondes, chaque sonde individuelle de l'ensemble étant spécifique de différentes parties du gènes HLA DR. Grâce au choix de multiples sondes correspondant aux séquences d'ADN des allèles connus, la spécificité du procédé de typage par oligonucléotides de la présente invention permet d'identifier tous les allèles des loci DRB1, DRB3 et DRBS. Bien entendu, le procédé de la présente invention pourrait être utilisé pour identifier les allèles d'autres loci extrêmement polymorphes tels que DQB1 et DPB1. Comme les différences alléliques sont essentiellement localisées dans l'exon codant pour le premier domaine des molécules HLA (aa 5-94), les sondes sont choisies pour être complémentaires de séquences spécifiques localisées dans cette région. Au cas ou de nouveaux allèles seraient découverts, ceux-ci sont immédiatement répertoriés dans un registre de séquences HLA classe II, qui permet d'actualiser la collection de traceurs informatifs et donc d'adapter la méthodologie à la détection de tout nouvel allèle.

Pour rationaliser le typage HLA classe II complet, on a proposé d'introduire tout d'abord une première étape du typage DR générique, lequel peut, avec un nombre limité de sondes, reconnaître les principales spécificités HLA-DR, c'est-à-dire HLA-DR1-DRw18. Cette étape est suffisante pour un grand nombre d'applications cliniques (voir B. Mach et J.M. Tiercy (1991) Human Immunol. 30, 278).

Sur la base des résultats de cette première étape, il est possible de choisir les sondes spécifiques nécessaires pour réaliser, dans un deuxième temps, un micropolymorphisme DRβ, pour détecter le polymorphisme DQB1 et si nécessaire pour caractériser les allèles DPB1.

L'analyse des spécificités HLA-DR1-DRw18 par la technique de typage par oligonucléotides peut être appliquée dans les laboratoires d'histocompatibilité pour le typage DR de routine, en remplacement de la sérologie DR, notamment pour effectuer le typage DR de patients en liste d'attente pour une greffe rénale ou le typage de donneurs de reins potentiels, le typage DR de patients leucémiques pour lesquels une greffe de moelle osseuse est envisagée, ainsi que des membres de leur famille ou des donneurs potentiels non apparentés, le typage DR à grande échelle pour la constitution de registres de donneurs de moelle volontaires, pour déterminer des associations entre des maladies et le système HLA, par exemple dans le cas de diabètes insulinodépendants, pour des applications en médecine prédictive ou encore pour des recherches de patemité et autres identifications judiciaires.

On donne ci-après quelques définitions de termes utilisés dans la présente demande :
"génotype" fait référence à l'ensemble des caractéristiques génotypiques d'un individu par opposition au "phénotype" qui sont les caractérisations d'un individu telles qu'elles ressortent de l'analyse des produits d'expression du gène et notamment des protéines.
"allèles" sont les différentes formes alternatives d'un même gène qui présentent des différences au niveau de la séquence nucléique. Ces différences se manifestent au niveau de l'ADN, de l'ARN et des protéines.
"polymorphisme" caractérise la diversité introduite dans une population par l'existence d'allèles différents pour un même gène. "oligonucléotide" tel qu'utilisé ici désigne les amorces, les sondes, les fragments d'acides nucléiques devant être détectés, etc... Les oligonucléotides peuvent être préparés par toute méthode appropriée connue.
"sonde nucléotidique" représente un fragment d'ADN ou d'ARN naturel, ou un oligonucléotide naturel ou de synthèse, ou un fragment d'ADN ou d'ARN de synthèse, non modifié ou comprenant une ou plusieurs bases modifiées telles que l'inosine (symbolisée par la lettre I), la méthyl-5-désoxycytidine, la diméthylamino-5-désoxyuridine, la désoxyuridine, la diamino-2,6-purine, la bromo-5-désoxyuridine ou toute autre base modifiée permettant l'hybridation.

L'invention sera mieux comprise à la lecture de la description détaillée qui va suivre, faite en référence à des exemples non limitatifs illustrant des modes de réalisation préférés du procédé de l'invention.

### EXEMPLE 1 :

Les ligands utilisés dans la présente invention et donnés ici à titre d'exemple, peuvent être des composés disponibles dans le commerce tels que dans le tableau 1 ci-après :

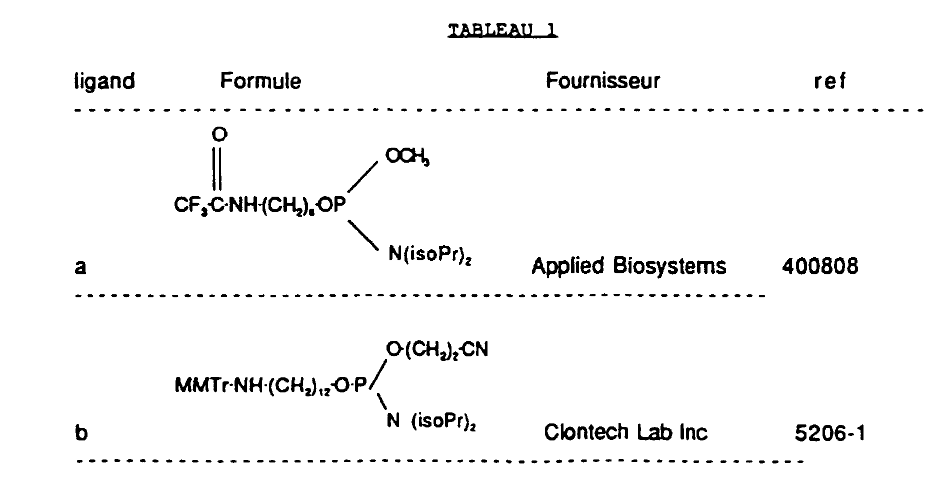

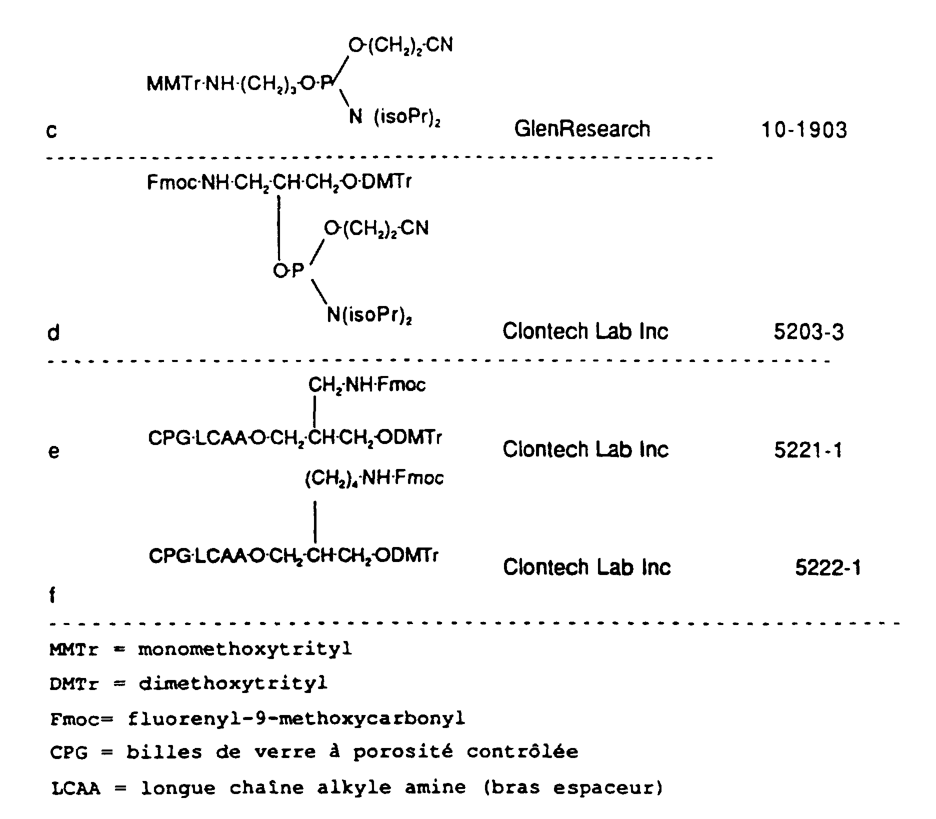

Le couplage d'un ligand phosphoramidite à un oligonucléotide est effectué selon le protocole général suivant :

Un oligonucléotide est synthétisé sur un appareil automatique 381 A de la société APPLIED BIOSYSTEMS en utilisant la chimie des phosphoramidites selon le protocole du constructeur. Le ligand phosphoramidite dissout dans de l'acétonitrile anhydre à une concentration de 0,2 M, est placé en position X du synthétiseur et l'addition du ligand se fait à l'extrémité 5' de l'oligonucléotide selon le protocole standard de la synthèse automatique lorsque la synthèse de l'oligonucléotide est achevée.

Dans le cas où le ligand est porteur d'un groupement protecteur dimethoxytrityle, tel que pour le composé d, il est nécessaire d'effectuer une étape supplémentaire de déprotection du groupement trityle par l'acide trichloroacétique en fin de synthèse.

Après déprotection une nuit à 55° C dans NH₄OH 33 %, suivie d'une précipitation dans de l'éthanol à - 20°C, l'oligonucléotide est séché sous vide et repris dans 1 ml d'H₂O.

Pour les composés référencés b et c, une étape supplémentaire de clivage du groupement monomethoxytrityle est effectuée selon le protocole du fabricant (CLONTECH et GLEN RESEARCH respectivement) après déprotection.

Dans le cas des composés portant la référence e et f, la synthèse automatique démarre par la silice greffée par le ligand selon le protocole standard. Le couplage ligand et oligonucléotide s'effectue par l'extrémité 3' de ce dernier.

Dans tous les cas, les oligonucléotides modifiés à leurs extrémités 5' ou 3' sont purifiés par chromatographie liquide haute pression (CLHP) en phase inverse sur une colonne Brownlee RP18 (10 mm - 25 cm).
- Conditions :: débit 4,6 ml/min
Gradient

10 % à 35 % de tampon B en 30 min.
35 % à 100 % de tampon B en 3 min.
Les caractéristiques des tampons A et B sont les suivantes.
Tampon A : 0.1 molaire Triethylammoniumacétate (TEAA) pH = 7,00
Tampon B : 50 % Tampon A + 50 % CH3CN.

### EXEMPLE 2 :

Couplage d'un oligonucléotide à l'Albumine de Sérum de boeuf (ASB).

Un oligonucléotide portant le bras aminolink 2 référencé a dans le tableau 1 est synthétisé tel que décrit dans l'exemple 1 : 3. 10⁻⁸ mole d'oligonucléotide sont séchés sous vide et repris dans 25 µl de tampon borate de sodium 0.1 M, pH 9.3. On additionne 500 µl d'une solution à 30 mg/ml de DITC (phénylène-1,4-diisothiocyanate Fluka 78480) dans le DMF. Le mélange est agité 1,5 h à température ambiante avant l'addition de 3 ml d'H₂O. Après extraction de la solution par le butanol (3 x 3 ml), la phase aqueuse restante (500 µl) est séchée sous vide puis reprise par 1,10⁻⁷ mole (6,6 mg) d'ASB (Pierce 30444) dans 400 µl de tampon borate (0,1 molaire pH 9.3). Après une nuit sous agitation à température ambiante, le conjugué est purifié par échange d'ions en CLHP sur une colonne AX300 (BROWNLEE 4.6 x 100 mm) par un gradient de NaCl (Tableau 1). Le pic du conjugué est dialysé contre de l'eau (2 x 1 litre) concentré sous vide, repris par 1 ml H₂O et stocké à - 20° C. Conditions chromatographiques :
- Gradient de :: 10 % B' à 56 % B' en 25 min.
56 % B' à 100 % B' en 2 min.

Les caractéristiques des tampons A' et B' sont les suivantes :
A' = 20 mM phosphate de sodium, pH 7,00, 20 % CH₃ CN.
B' = Tampon A' + 1 M NaCl ou 2M NaCl.

### EXEMPLE 3:

On a représenté dans le tableau 2 les alignements d'acides aminés des différents allèles du gène DR Beta dans le but de définir les positions des acides aminés mutés par rapport à la séquence consensus choisie (appelée "DR CONS"). Ces mutations correspondent à des mutations non silencieuses au niveau de l'ADN c'est à dire des mutations qui induiront un changement d'acide aminé. On sait en effet que les acides aminés sont codés au niveau de l'ADN par des triplets de bases. Une mutation sur la troisième position n'entrainera généralement pas de changement d'acide aminé. Par contre le changement de la seconde base induira assez souvent un changement d'acide aminé. Enfin, une mutation sur la première base entraînera toujours une modification de l'acide aminé.

Dans le cas du typage des différents allèles, on utilise donc le plus souvent les mutations sur l'ADN correspondant aux mutations non silencieuses. Mais il est possible de détecter une mutation de type silencieuse, par exemple dans le but de différencier 2 allèles très proches.

Dans le tableau 3, on a représenté les alignements des nucléotides du gène DRBeta pour tous les allèles connus et publiés dans la littérature à ce jour, par rapport à la même séquence consensus que dans le tableau 2.

La nomenclature utilisée pour désigner les différents allèles est celle proposée lors de la cinquième conférence d'histocompatibilité (Leiden, Hollande, 1991). Les indications entre parenthèses dans le tableau 2 représentent la nomenclature précédente.

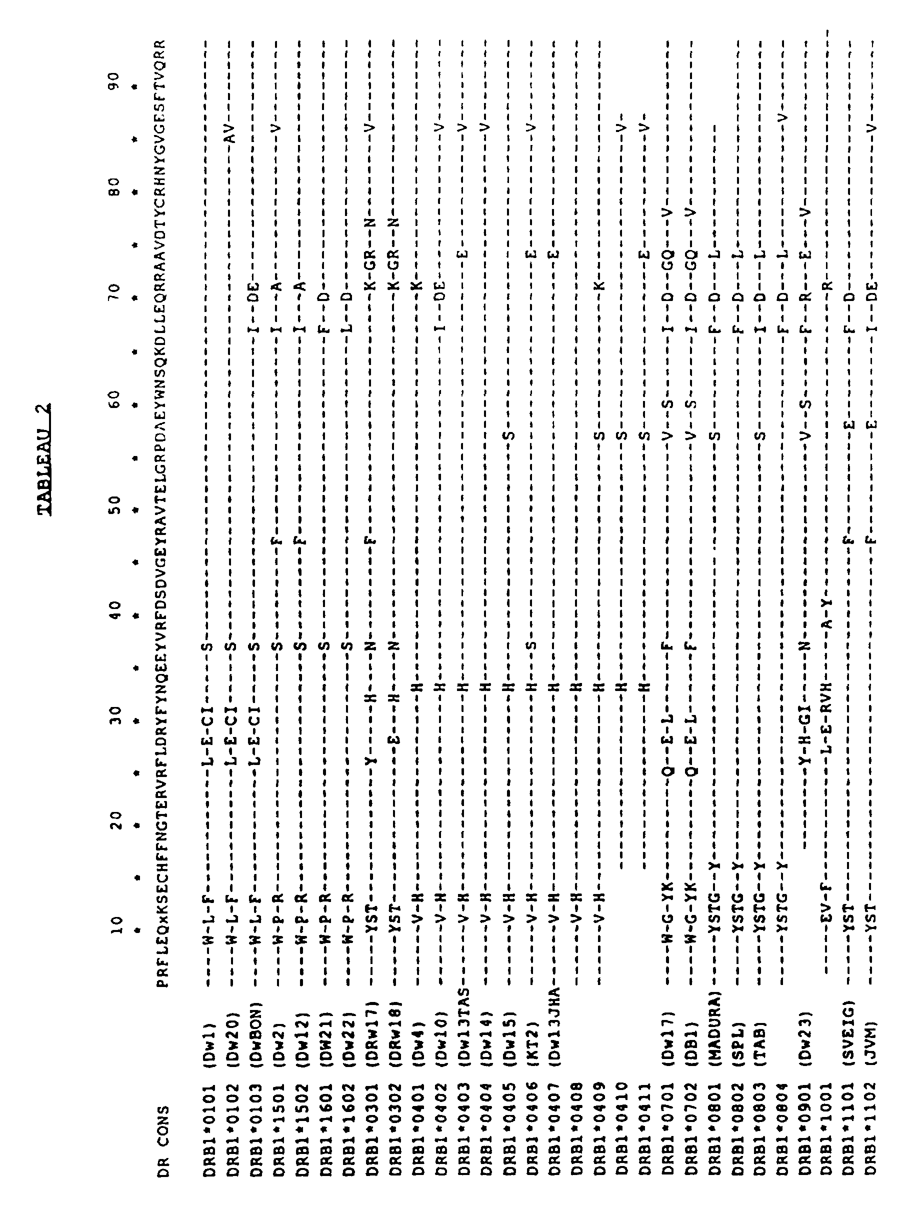

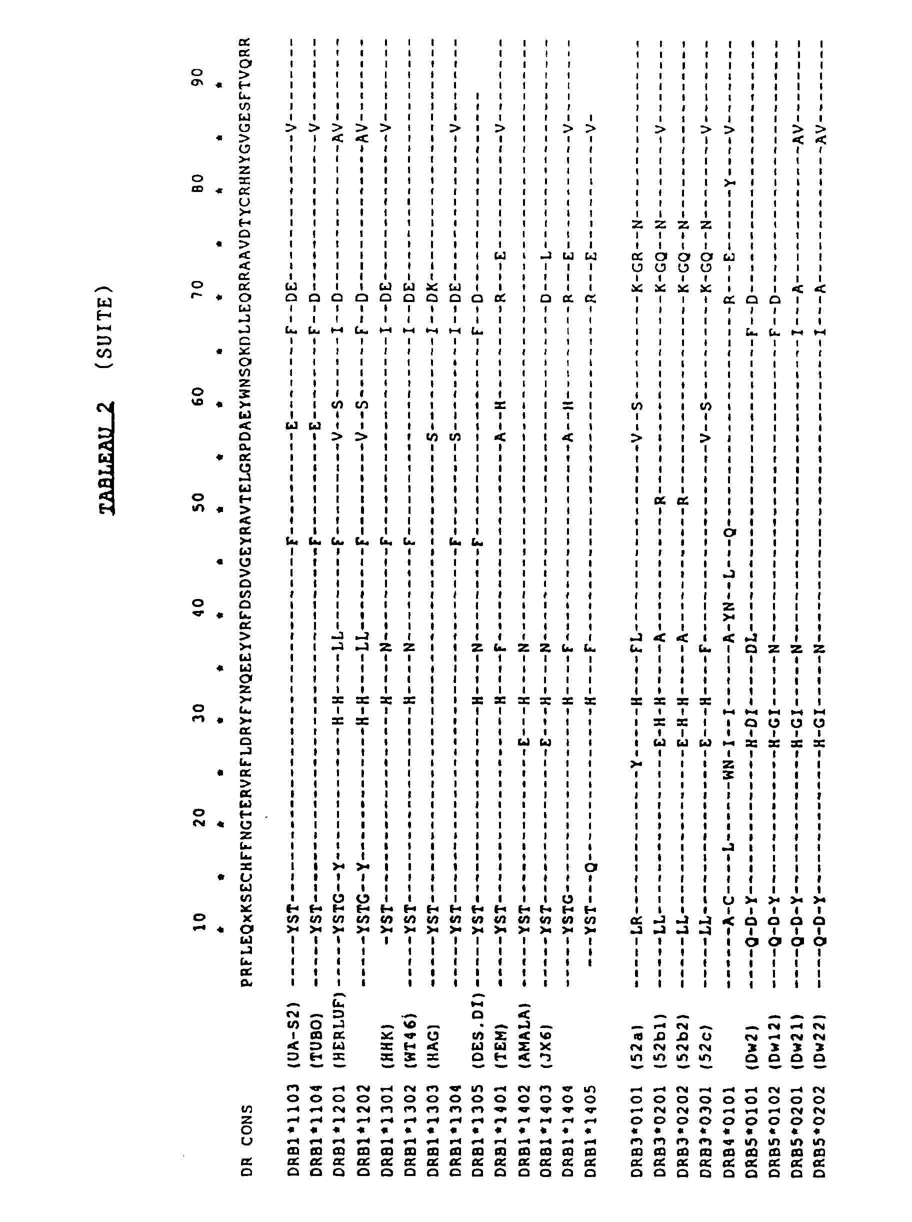

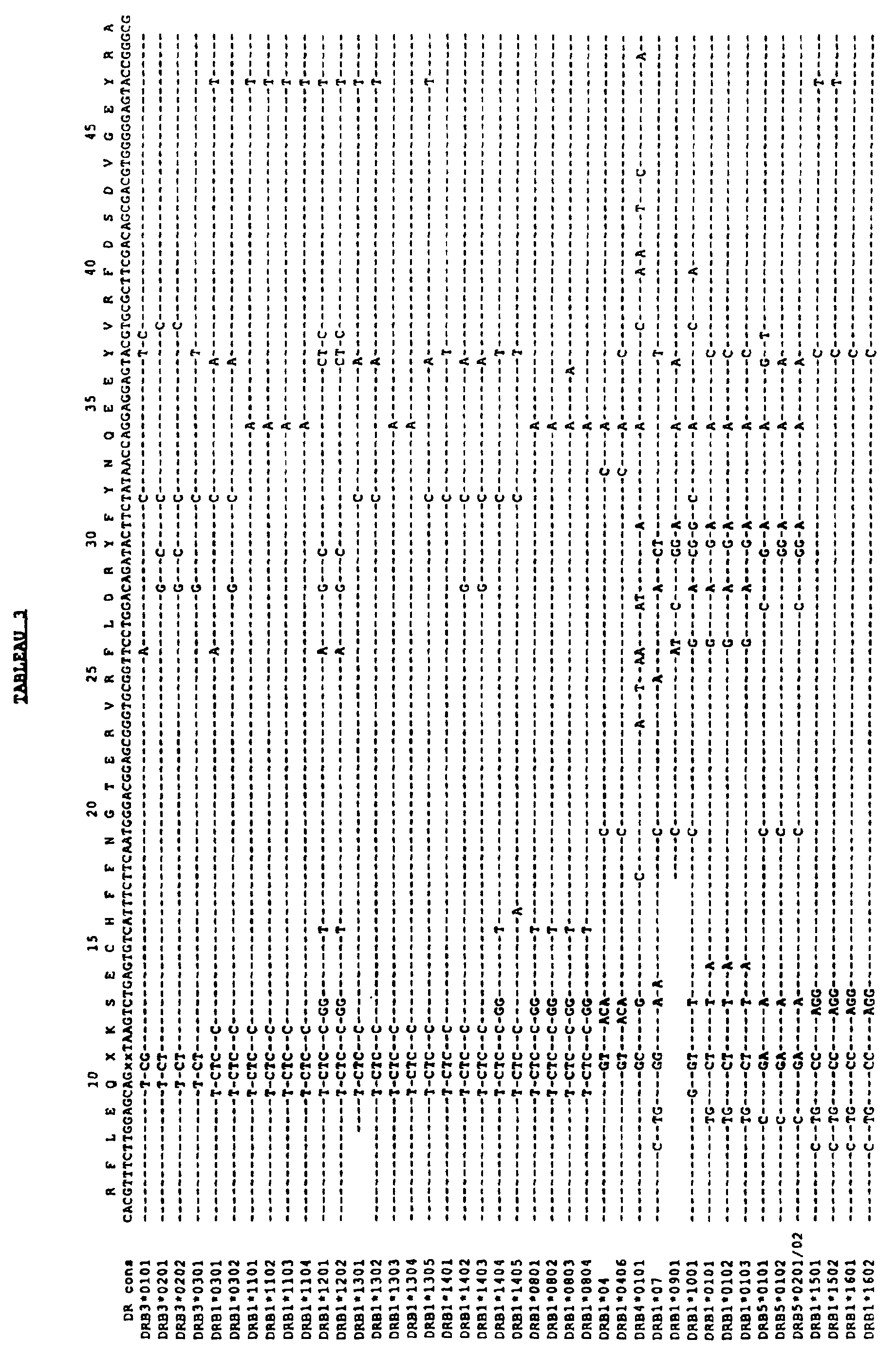

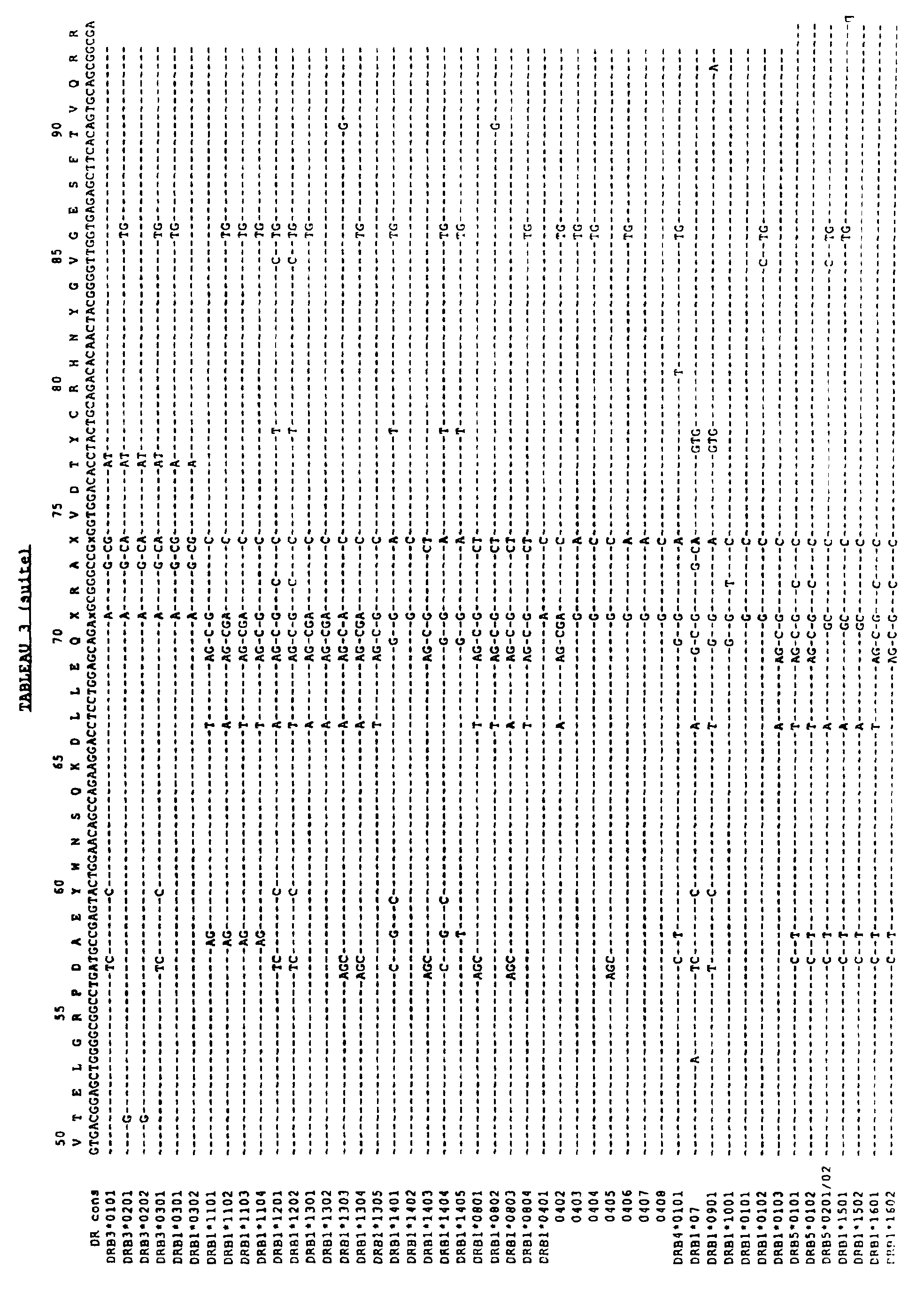

### EXEMPLE 4:

En utilisant les 2 protocoles décrits précédemment on a synthétisé des oligonucléotides portant soit un ligand, tel que décrit dans l'exemple 1 et qui sont résumés dans le tableau 4, soit couplé à l'ASB, tel que décrit dans l'exemple 2 et qui sont résumés dans le tableau 5

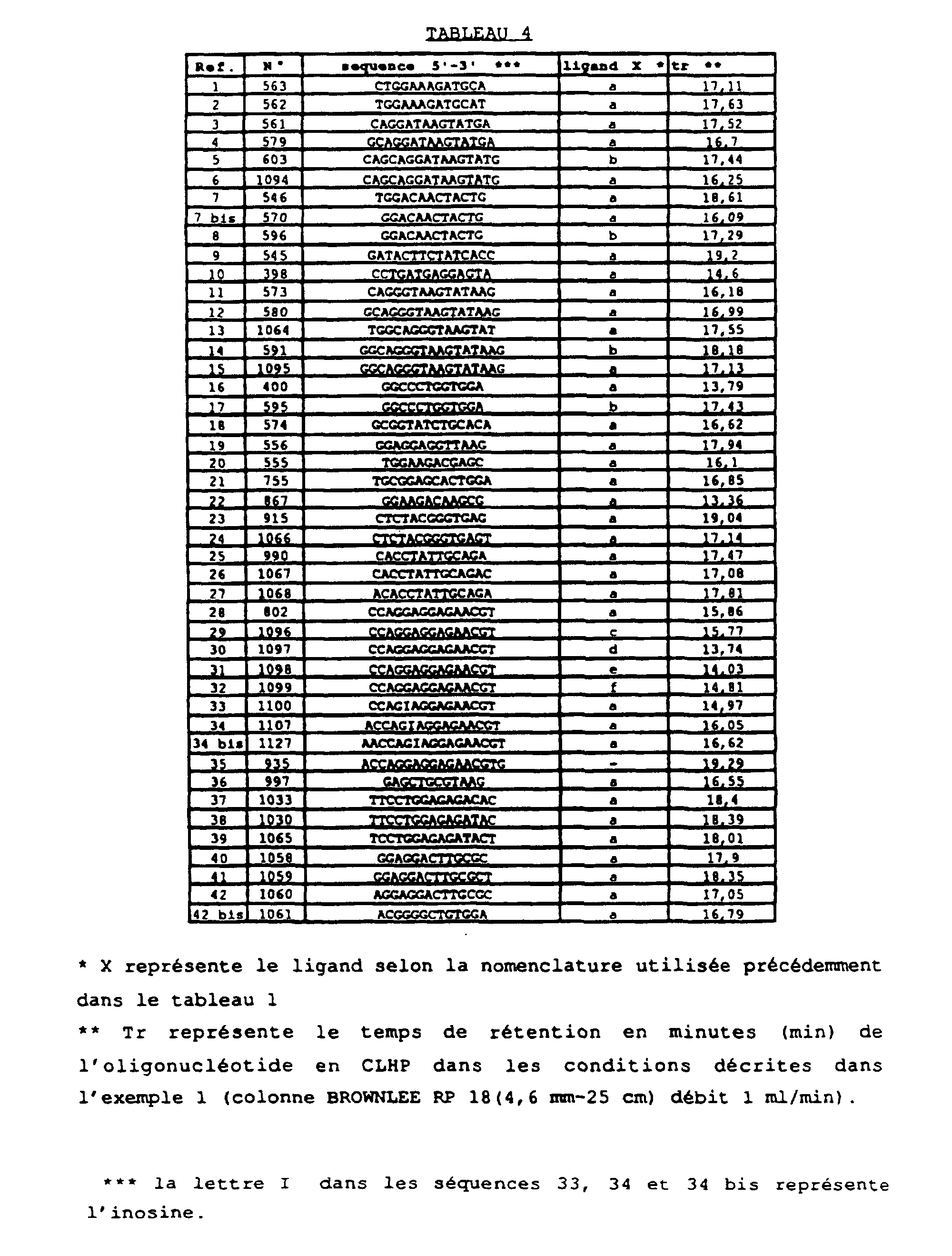

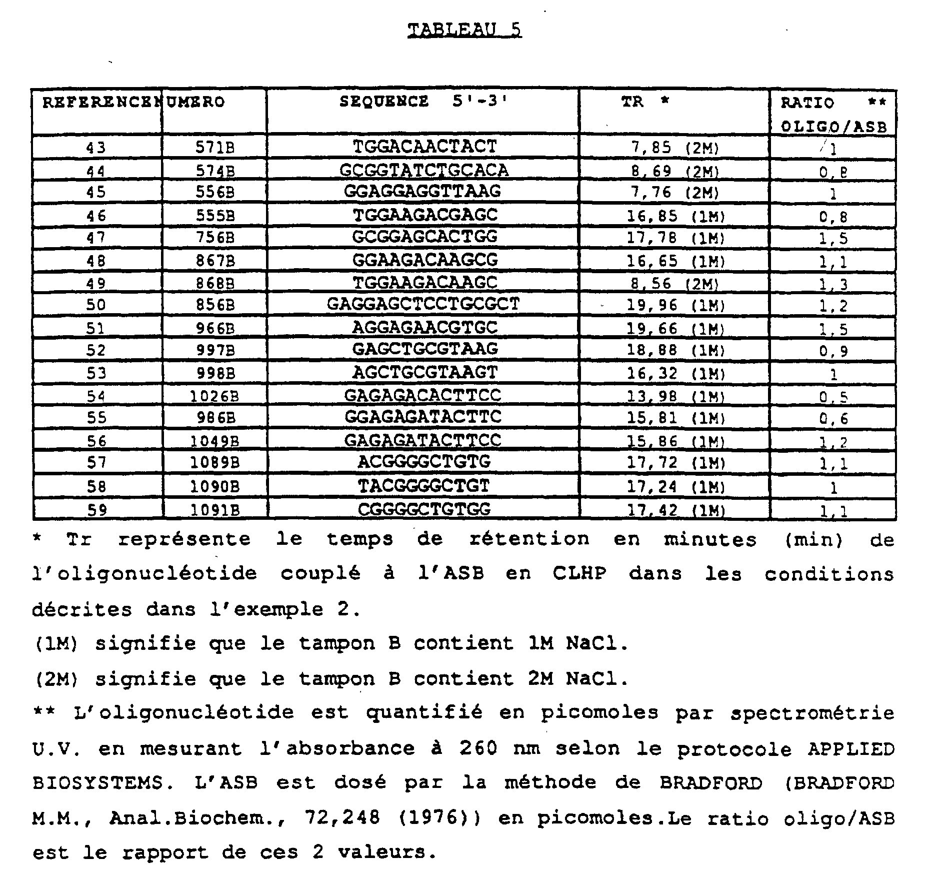

On a défini dans cet exemple des oligonucléotides de capture que l'on peut synthétiser, sans ligand, avec un ligand ou bien coupler par exemple à l'ASB. Le choix des séquences des oligonucléotides synthétisés tient compte de l'alignement des séquences d'ADN des différents allèles décrits dans le tableau 3 de l'exemple 3. Les sondes oligonucléotidiques sélectionnées, utilisées par exemple comme sondes de capture, permettent d'effectuer un plan de typage comme décrit dans le tableau 6.

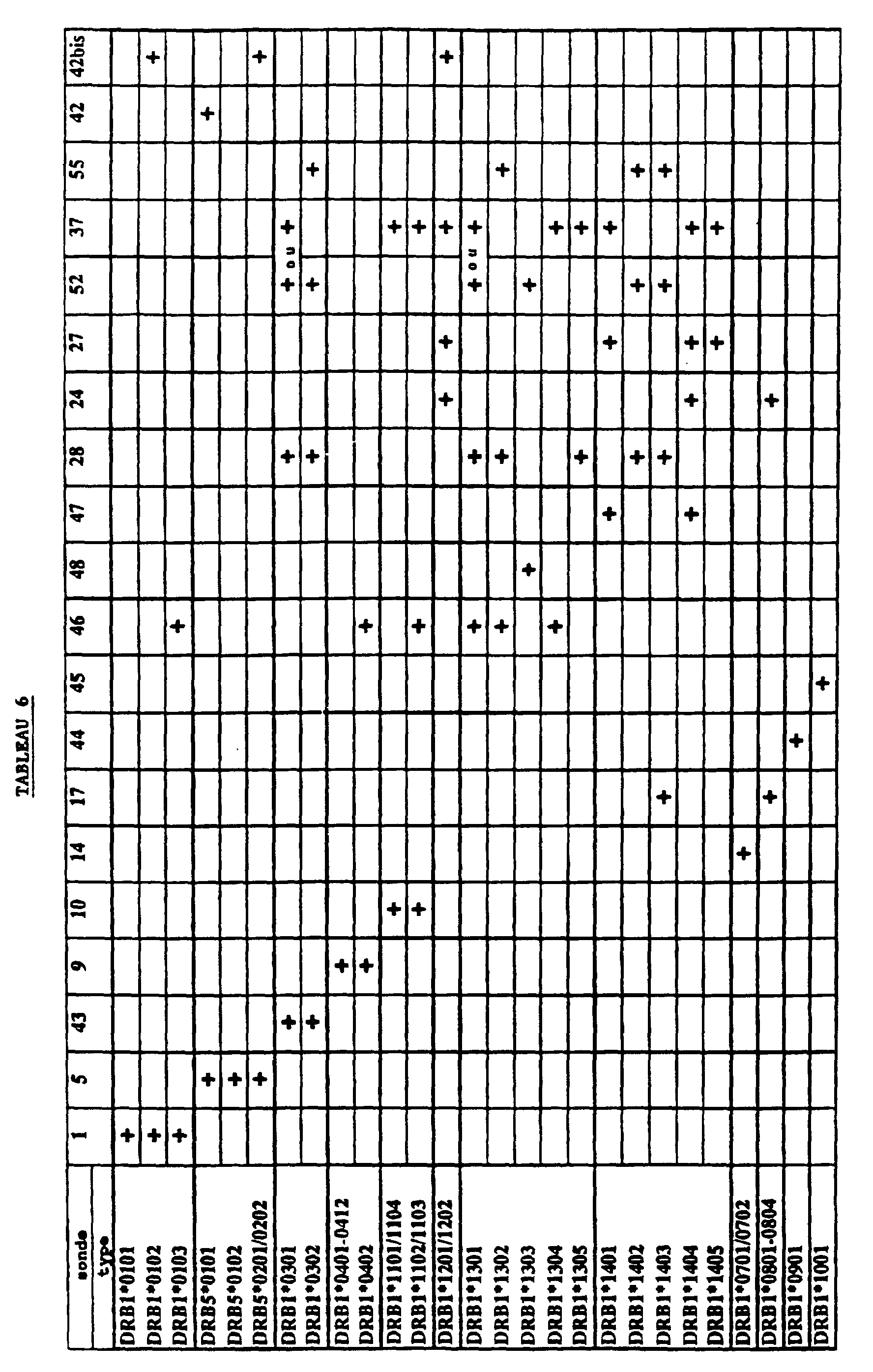

Dans le tableau 6, les indications entre parenthèses représentent la nomenclature utilisée avant la conférence d'histocompatibilité (1991) des sous types de l'allèle DRBS.

Le signe + signifie que le sous type de la ligne considérée du tableau 6 donne une hybridation avec la sonde de la colonne correspondante.

A l'aide du tableau 6, il est possible d'interpréter facilement les résultats obtenus (hybridation ou absence d'hybridation) avec diverses sondes par exemple une cible donnant une réponse positive avec les sondes 43, 14, 28 et 37 correspond aux types DRB1*0301/DRB1*07.

### EXEMPLE 5: préparation des sondes de détection

Selon l'exemple 2 l'oligonucléotide activé et séché sous vide est repris par 1,25 10⁻⁷ mole (5 mg) de peroxydase de raifort (BOEHRINGER MANHEIM 413470) dans 200 µl de tampon borate de sodium 0,1M, pH 9,3.

Le protocole de purification est identique : le conjugué est stocké à -20°C dans un tampon 50 mM tris HCI, pH 7,0 ,40% glycérol.

Le tableau 7 résume les différents conjugués utilisés pour la détection HLA DR.

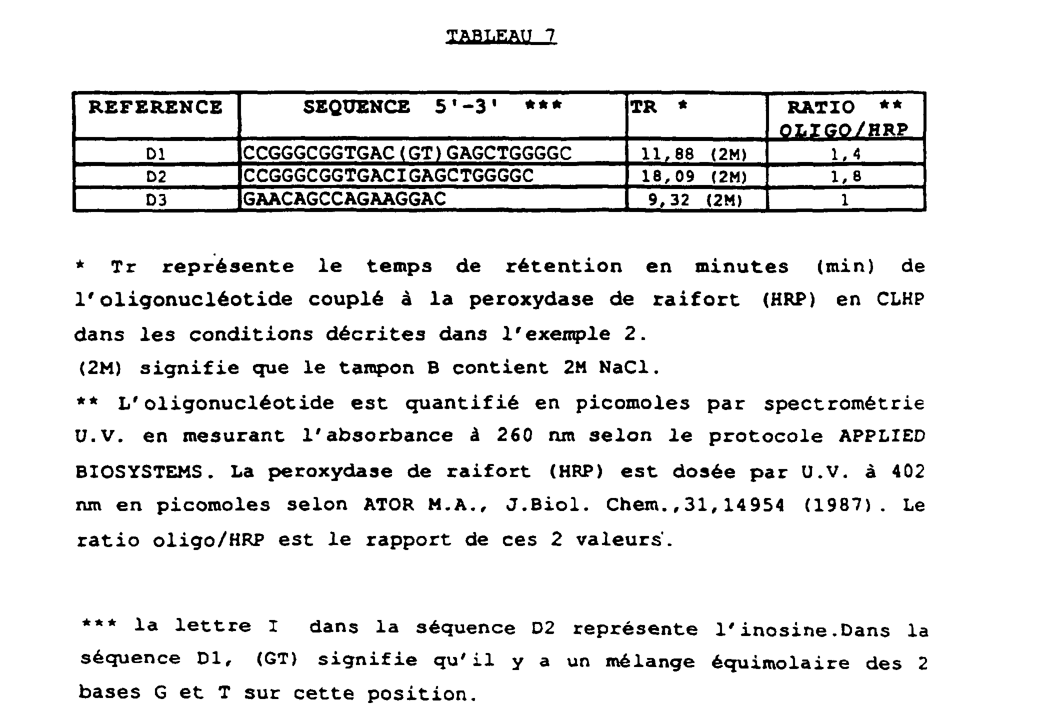

### EXEMPLE 6 : préparation du matériel génétique

L'extraction d'acides nucléiques à partir de sang total est effectuée dans un appareil Applied Biosystems d'après le protocole suivant : 2 à 6 ml de sang total sont repris dans du tampon TE (10 mM Tris-HCI pH 8,00, 1 mM EDTA) (quantité suffisante pour 6 ml) et sont déposés dans une ampoule d'extraction de 30 ml. Une solution de protéinase K (840 unités dans 20 mM Tris-HCI pH 8,5) est ajoutée. L'ensemble est incubé sous agitation 1 heure à 55°C. L'excès de protéines présentes est éliminé par 2 extractions simultanées (8,5 ml) par un mélange de phénol chloroforme. L'ensemble est agité pendant 20 minutes à 60°C. Après élimination de la phase organique une nouvelle extraction phénolique est effectuée. L'excès de phénol est éliminé par une extraction au chloroforme (9,5 ml), 10 minutes à 37°C. L'ADN contenu dans la phase aqueuse est précipité par addition de 0,5 ml d'acétate de sodium 3 M pH 5,5 et 13,5 ml d'isopropanol puis récupéré sur un filtre. L'ADN est ensuite repris dans 1 ml d'eau distillée, puis dosé en spectrophotométrie à 260 nm.

### EXEMPLE 7 : amplification de l'ADN

L'Amplification enzymatique est effectuée par la technique de réaction en chaîne par la polymérase (PCR) (MULLIS et FALOONA, Meth. in Enzymol. vol. 155, pp 335-350) d'après le protocole suivant :

Dans un tube de type Eppendorf sont ajoutés 0,1 à 2 µg d'ADN purifié ou non dans un volume total de 100µl du tampon suivant :
- 10µl de tampon de PCR 10 fois concentré (500 mM KCl, 100 mM Tris-HCI pH 8,3 (20°c), 15 mM MgCl₂, 0,1% gélatine)
- 2 µl dNTP (dATP, dCTP, dGTP, TTP) 0,5 µM
- 2 µl de chaque amorce correspondant à 25 pmoles
- 1,5 unités de Taq polymerase (Perkin Elmer Cétus)
- eau distillée (quantité suffisante pour 100 µl)
- 50 µl d'huile de paraffine

Le tube est déposé dans un Thermocycler (Perkin Elmer Cétus) dans lequel seront effectués les 35 cycles de températures suivants:
- 0,5 minute de dénaturation à 95°C
- 0,5 minute d'hybridation à 55°C
- 0,5 minute d'élongation à 72°C
Les amorces utilisées ont la séquence suivante :

amorce 1 = 5'-CCGGATCCTTCGTGTCCCCACAGCACG-3'

amorce 2 = 5'-TCGCCGCTGCACTGTGAAG-3'

### EXEMPLE 8 :

Dans un puits d'une plaque de microtitration en polystyrène (Nunc 439454) sont déposés 100 µl d'une solution d'un oligonucléotide de capture d'une spécificité DR donnée à une concentration de 0,15 µM dans du PBS 3 x (0,45 M NaCI, 0,15 M phosphate de sodium pH 7.0). Il y a autant de puits remplis que nécessaires pour le typage.

Dans tous les cas un contrôle positif doit être ajouté dans le but de vérifier l'efficacité de l'étape d'amplification ainsi que l'étape de détection. La sonde de capture qui est utilisée comme contrôle positif est présente sur tous les allèles connus à ce jour et a la séquence suivante :

5'- GGGGAGTACCGGGCGGTGACGGAGCTGGGGCGGCCT - 3'

La plaque est lavée 3 fois avec 300 µl de PBS Tween (0,15 M NaCl, 0,05 M phosphate de sodium, pH 7,0 ; 0,5 % Tween 20 (Merck 822184)). Le produit d'amplification (100 µl) tel que décrit dans l'exemple 7 est dénaturé par 10 µl de NaOH 2 N pendant 5 minutes sous agitation à température ambiante. 10 µl d'acide acétique 2N puis un volume de tampon PEG (Phosphate de sodium 0,1 M, pH 7,0, NaCl 0,5 M, Tween 20 0,65 %, ADN de sperme de saumon (Sigma D 9156) 0,14 mg/ml, PEG 4 000 (Merck 807490) 2 %) équivalent à n x 50 µl (n étant le nombre de sondes de capture nécessaires au typage) sont additionnées successivement à cette solution. 50 µl de cette solution sont répartis par puits suivis de 50 µl de la sonde de détection (conjugué oligonucléotide péroxydase) à une concentration de 15 nM dans le tampon PEG. La plaque est incubée 1 h à 37° C et lavée par 3 x 300 µl de PBS Tween. 100 µl de substrat OPD (orthophenylenediamine Cambridge Medical Biotechnology ref/456) dans un tampon OPD (0,05 M acide citrique, 0,1 M Na₂HPO₄, pH 4,93) à la concentration de 4 mg/ml auquel on ajoute extemporanément H₂O₂ à 30 volumes au 1/1000, sont ajoutés par puits. Après 20 min de réaction, l'activité enzymatique est bloquée par 100 µl d'H₂SO₄ 1N et la lecture est effectué sur Axia Microreader (bioMérieux) à 492 nm.

### EXEMPLE 9:

6 ADN, préparés selon la méthode décrite dans l'exemple 6, sont amplifiés selon la méthode décrite dans l'exemple 7.

Le protocole de typage comporte les sondes de capture suivantes:

5' a-GATACTTCTATCACC 3' = oligonucléotide de spécificité DR 3 portant le ligand a en 5' (référencé 545 )

5' GATACTTCTATCACC 3' = oligonucléotide de séquence identique mais sans ligand (référencé 545 nu)

5' a-TGGACAACTACTG 3'= oligonucléotide de spécificité DR 4 portant le ligand a en 5' (référencé 546 )

5' TGGACAACTACTG 3' = oligonucléotide de séquence identique mais sans ligand (référencé 546 nu)

Le protocole de typage est conforme au protocole général décrit dans l'exemple 8.

Les sondes D1 et D2 (tableau 7) sont utilisées en mélange 50%-50% comme sondes de détection.

Les résultats sont présentés dans le tableau 8 ci-après :

**TABLEAU 8**

| **ADN** | **TYPAGE** | **DR3 545 NU** | **DR3 545** | **DR4 546 NU** | **DR4 546** |
|---|---|---|---|---|---|
| **1** | **DR11/DR11** | 0,019 | 0,025 | 0,021 | 0,025 |
| **2** | **DR4/DR4** | 0,018 | 0,021 | 0,021 | 0,138 |
| **3** | **DR8/DR7** | 0,017 | 0,022 | 0,019 | 0,019 |
| **4** | **DR3/DR11** | 0,026 | 0,423 | 0,021 | 0,027 |
| **5** | **DR3/DR4** | 0,023 | 0,176 | 0,026 | 0,296 |
| **6** | **DR3/DR3** | 0,023 | 0,387 | 0,023 | 0,018 |

Les 2 sondes de capture sans ligand ne différencient pas les spécificités des ADN alors que les mêmes séquences avec le ligand a permettent d'identifier les spécificités DR2 et DR4 des ADN.

### EXEMPLE 10:

24 ADN, préparés selon la méthode décrite dans l'exemple 6, sont amplifiés selon la méthode décrite dans l'exemple 7. Le protocole de typage est conforme au protocole général décrit dans l'exemple 8.

Les sondes D1 et D2 (tableau 7) sont utilisées en mélange 50%-50% comme sondes de détection.

Le protocole de typage comporte les sondes de capture récapitulées dans le tableau 9 ci-après:

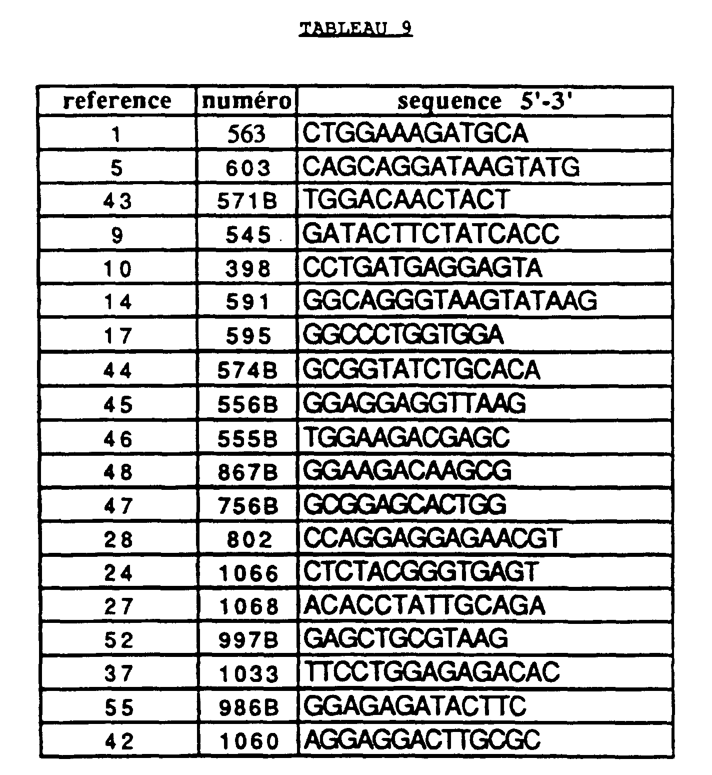

Les résultats du typage sont donnés dans la tableau 10:

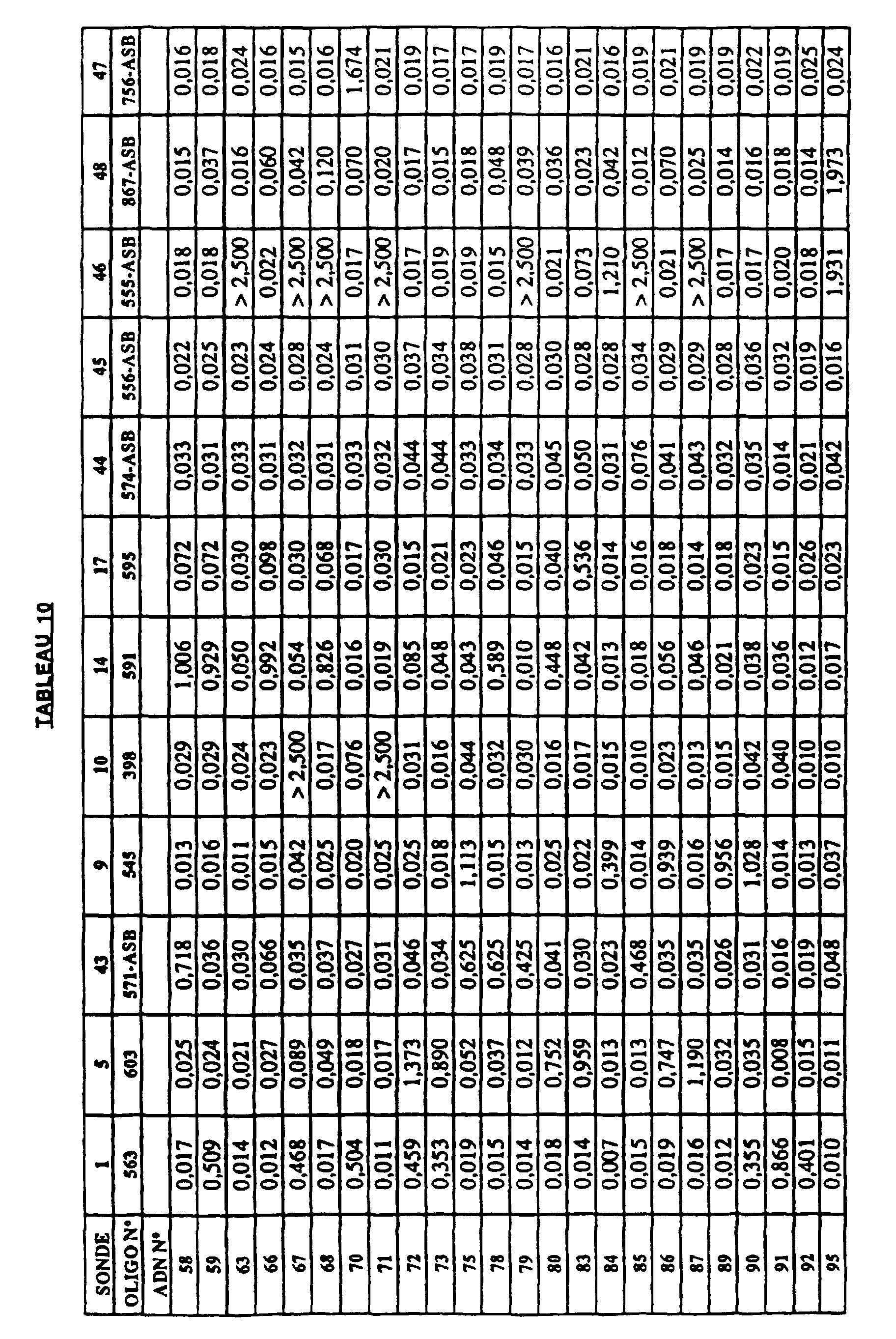

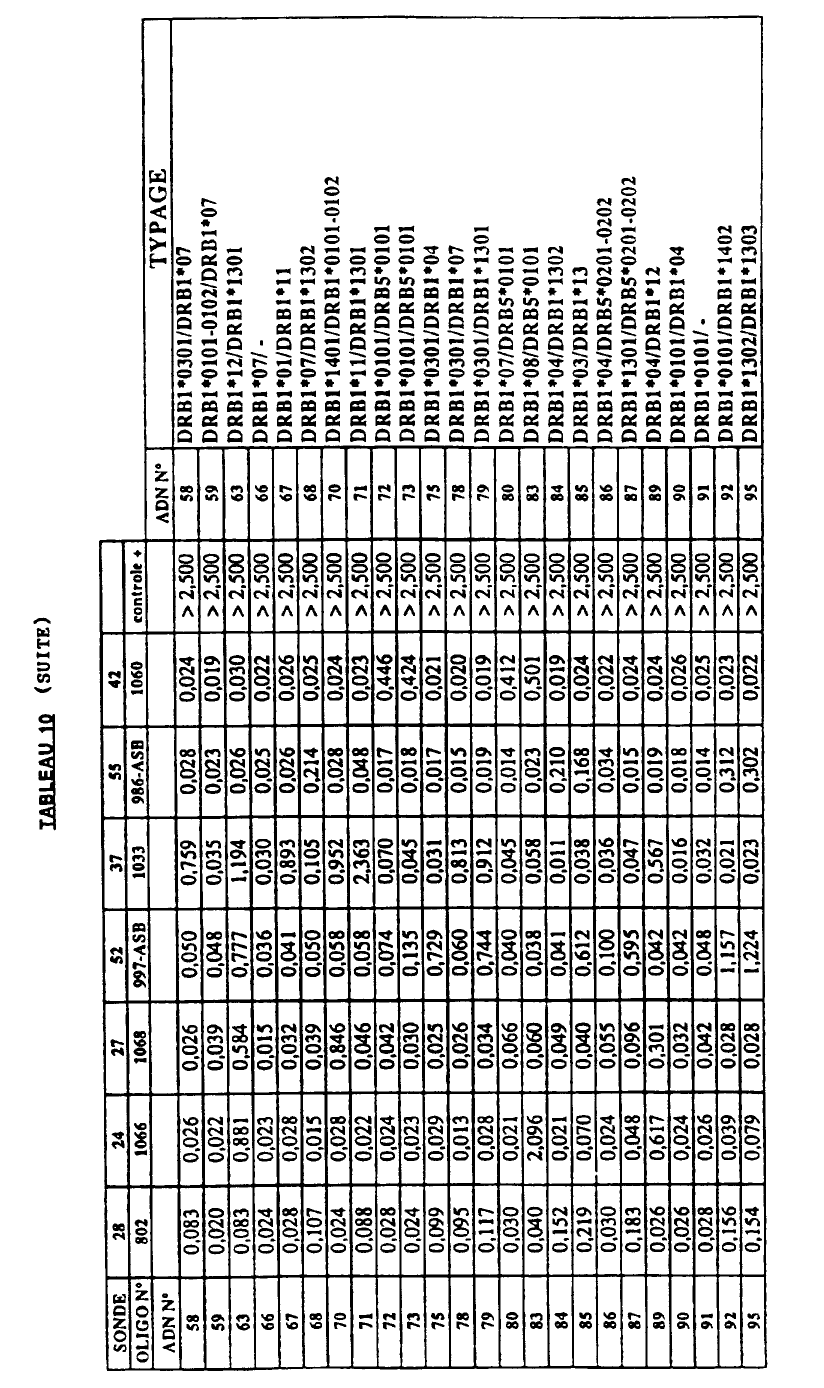

La méthode décrite nous permet de typer sans ambiguïté les 24 ADN testés.

### EXEMPLE 11:

La température préférentielle d' hybridation pour le typage HLA DR décrit dans la présente invention est 37 °C. Il est cependant possible de modifier cette température d'hybridation .

L'exemple suivant est identique à l'exemple 10 à l'exception de la température d'hybridation qui a été modifiée de 37°C à 45°C. Le typage est réalisé sur 11 ADN.

Les sondes de capture utilisées sont données dans le tableau 11 ci-après:

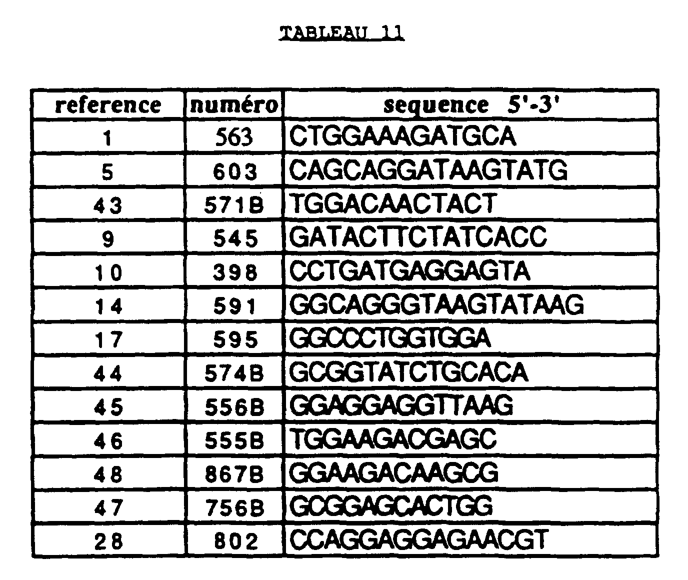

Les résultats du typage sont donnés dans le tableau 12 ci-après:

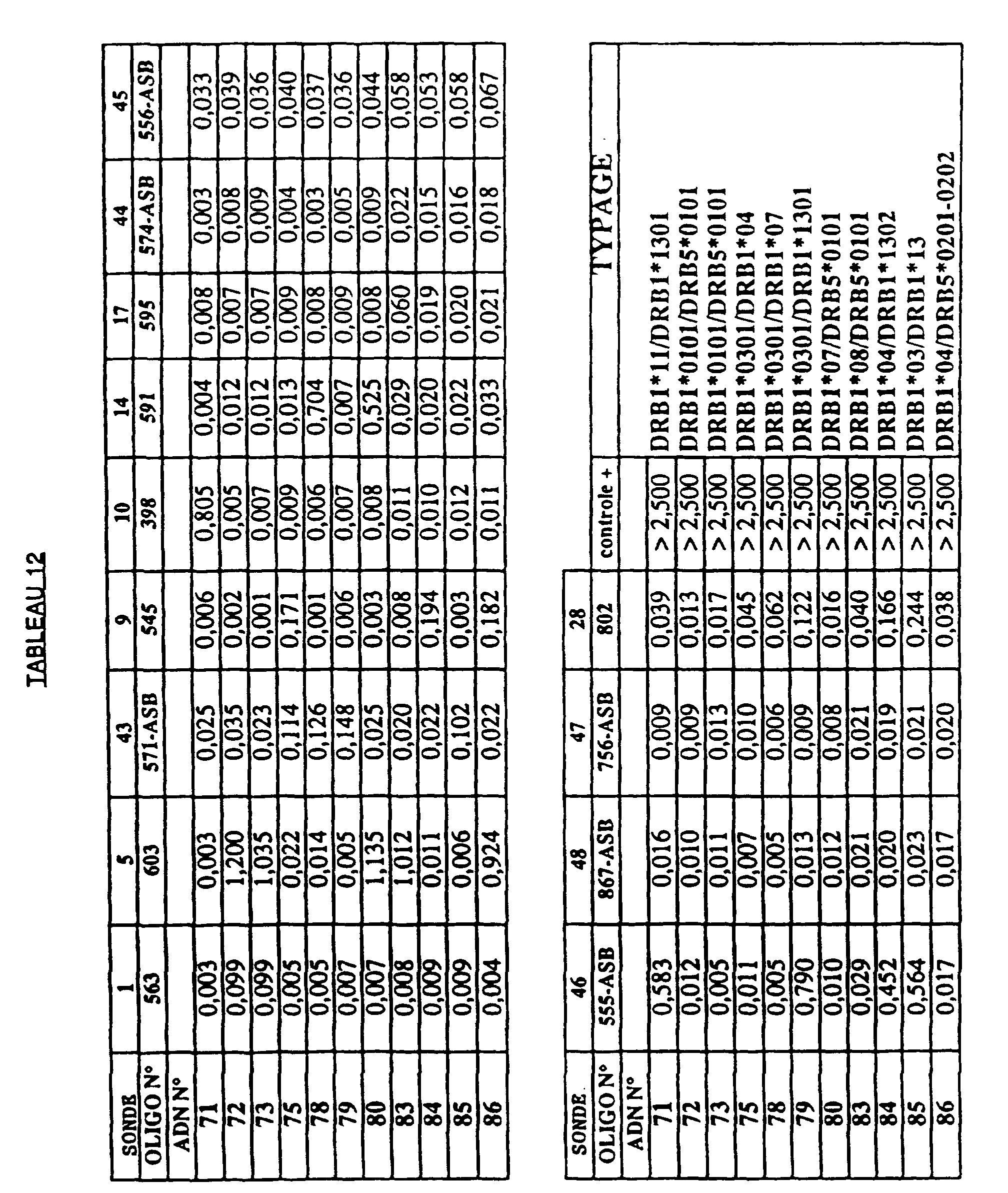

### EXEMPLE 12:

Le tampon d'hybridation préférentiel nommé tampon PEG utilisé pour le typage HLA DR comme décrit dans l'exemple 8 a la composition suivante : Phosphate de sodium 0,1 M, pH 7, NaCI 0,5 M, Tween 20 0,65 %, ADN de sperme de saumon (Sigma D 9156) 0,14 mg/ml, PEG 4000 (Merck 807490) 2 %).

On a utilisé le même tampon contenant de la formamide (10% final). Il est connu que la formamide permet de diminuer la température d'hybridation.

Si l'on effectue toujours l'hybridation à 37°C en présence de formamide, on doit donc augmenter la spécificité de la détection.

Le typage est réalisé sur 24 ADN qui sont ceux qui ont été utilisés dans l'exemple 10.

Les sondes de capture utilisées et les valeurs obtenues sont données dans le tableau 13 ci-après:

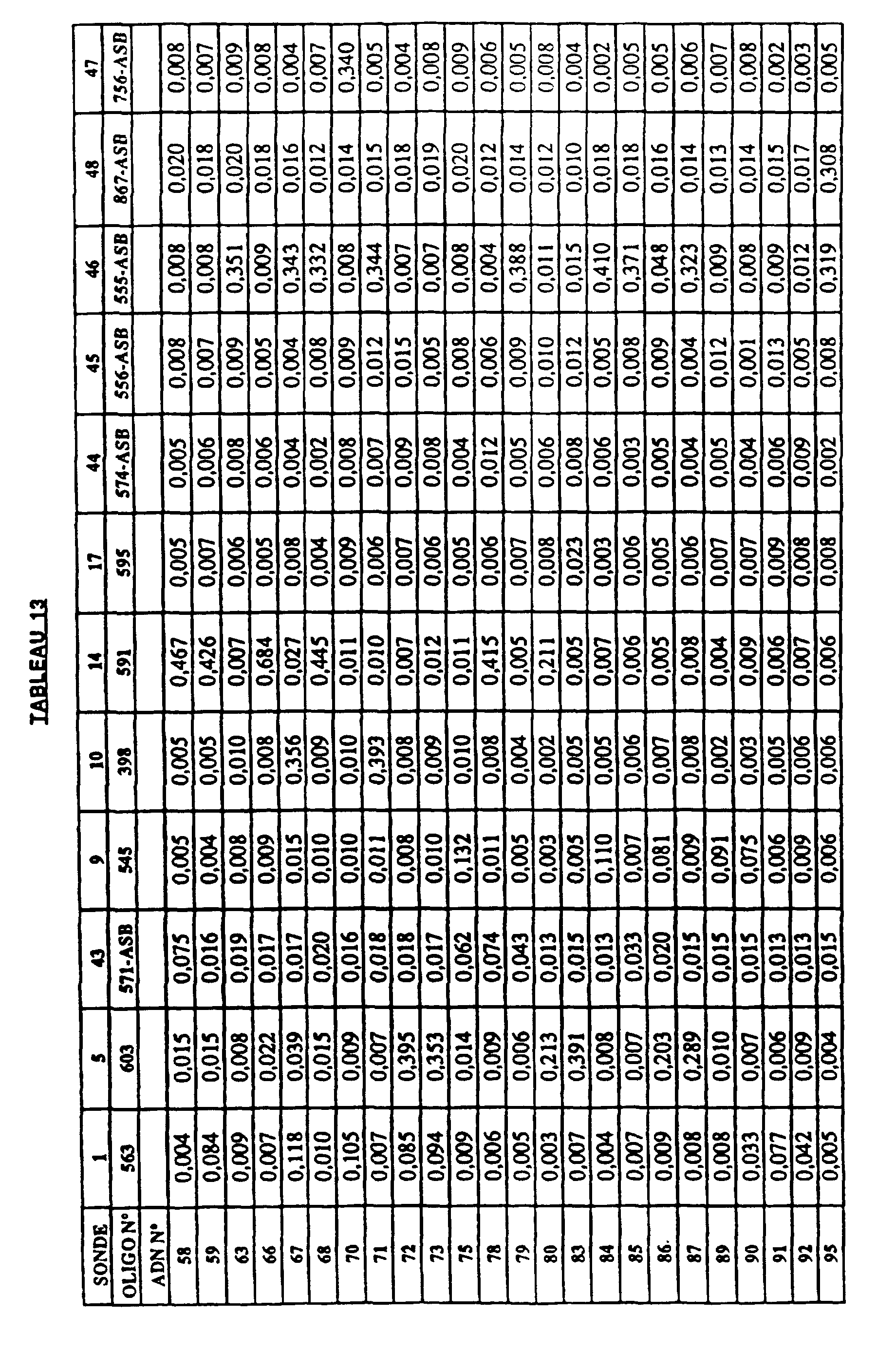

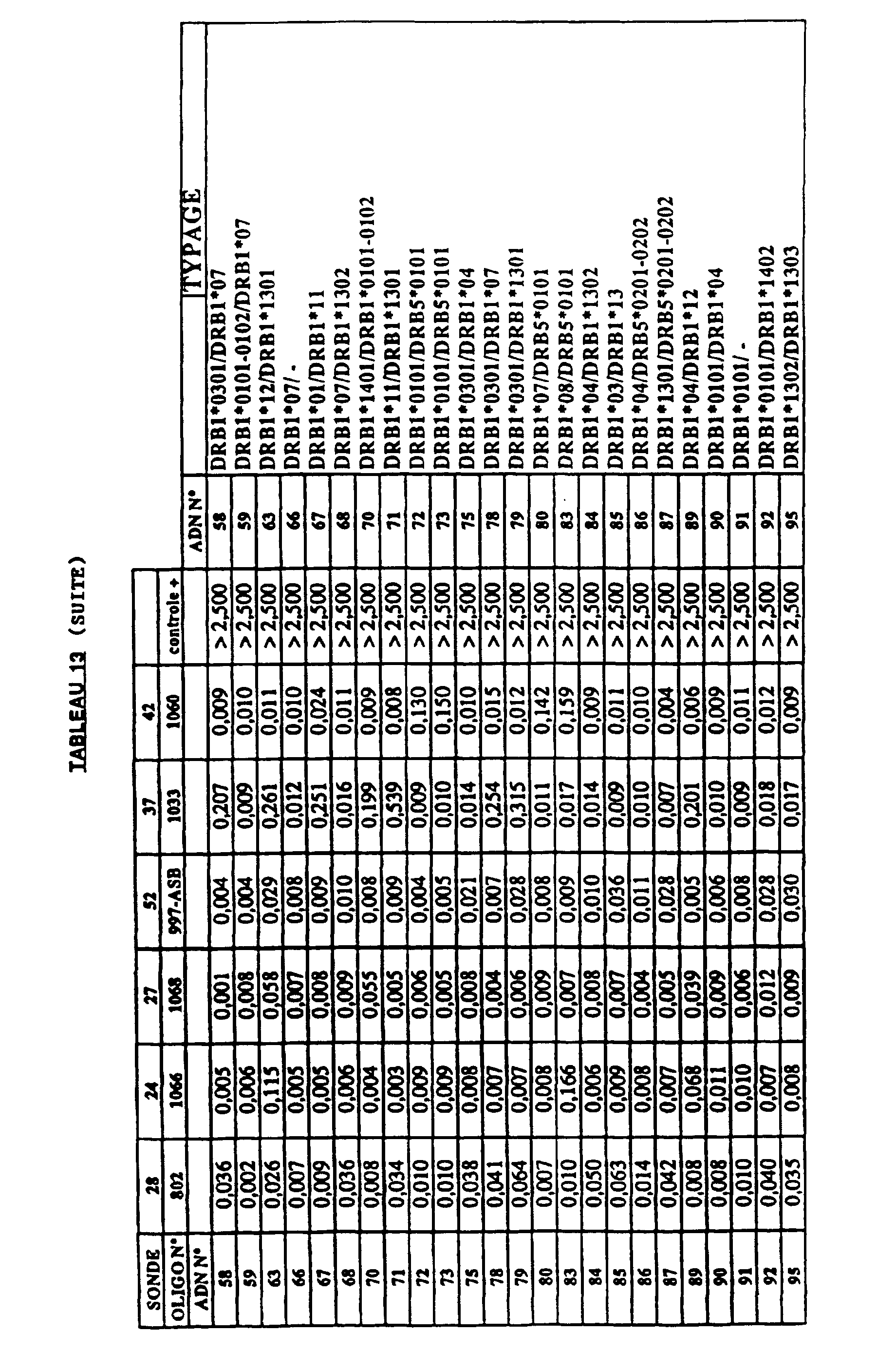

La température préférentielle d'hybridation est 37°C et le tampon d'hybridation préférentiel est le tampon PEG, mais comme le montrent les résultats de l'exemple 11 et 12, on voit qu'il est possible de faire varier à la fois la température d'hybridation et le tampon d'hybridation.

Comme cela ressort de la description ci-avant, le procédé de la présente invention combine les avantages pratiques suivants : une spécificité optimale avec discrimination possible de tous les allèles,
une simplicité d'exécution et un coût réduit par rapport à l'analyse sérologique,
une rapidité d'exécution avec obtention des résultats 90 minutes environ après amplification, soit une durée totale inférieure à 12 heures, ce qui est essentiel pour les donneurs de reins,
une compatibilité au typage individuel essentielle pour les typages d'urgence et l'utilisation en petits laboratoires, un signal quantifiable par mesure de Densité Optique et un traitement éventuel des résultats par un système informatique simple, et une adaptabilité à des systèmes automatiques.

## Revendications

1. Ensemble de sondes nucléotidiques permettant de déterminer selon les techniques usuelles de typage par oligonucléotides, les types HLA DR et/ou sous types associés, ledit ensemble de sondes comprenant au moins les sondes nucléotidiques suivantes, ou leurs complémentaires (la partie soulignée correspondant à la séquence optimum, et la lettre I représentant l'inosine):
(a): 
(G)CTGGAAAGATGCA(T) , CAGCAGGATAAGTATG(A) , (G)TGGACAACTACT(G),
GATACTTCTATCACC(AA) , (G)CCTGATGAGGAGTA(C) ,
(G)GGCCCTGGTGGA(CA) , (T)GCGGTATCTGCACA , GGAGGAGGTTAAG(TT) ,
(T)GGAAGACAAGCG(G) , (T)GCGGAGCACTGG(A),
(A)CCAGIAGGAGAACGT(G) , (A)CCAGGAGGAGAACGT(G) ,
(A)CTCTACGGGTGAGT(G) , (G)ACACCTATTGCAGA(C)
(b): (C) TGGAAGACGAGC (G) , et
(c) (T) GGCAGGGTAAGTATAAG,
les bases entre parenthèses étant facultatives,
lesdites sondes pouvant être sous forme marquée, ou couplées de façon covalente à un ligand facilitant leur fixation sur un support solide, ou fixées sur un support solide.

2. Ensemble de sondes selon la revendication 1 comprenant en outre les sondes suivantes (la partie soulignée correspondant à la séquence optimum) (G)GAGCTGCGTAAG(T) , TTCCTGGAGAGACAC_, (G)GGAGAGATACTTC(C) et/ou les sondes suivantes

(G) AGGAGGACTTGCGC (T) , (T) ACGGGGCTGTGGA (G) .

3. Ensemble de sondes selon la revendication 1 ou 2 **caractérisé par le fait qu'**il contient la sonde suivante:

AACCAGIAGGAGAACGT.

4. Ensemble de sondes salon l'une quelconque des revendications précédentes, **caractérisé par le fait que** lesdites sondes sont couplées de façon covalente avec un ligand, en particulier avec un ligand comprenant au moins un groupement fonctionnel polaire.

5. Ensemble de sondes selon la revendication 4, **caractérisé par le fait que** ledit groupement fonctionnel polaire est un groupement aminé.

6. Ensemble de sondes selon l'une des revendications 4 à 5, **caractérisé par le fait que** ledit ligand comprend une partie hydrophobe.

7. Ensemble de sondes selon l'une quelconques des revendications 4 à 6, **caractérisé par le fait que** ledit ligand comprend un groupement de formule générale (I) 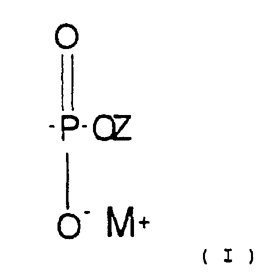 dans laquelle Z représente un radical alkyle ou alcényle de 2 à 12 atomes de carbone, linéaire ou ramifié, non substitué ou substitué par un ou plusieurs groupements hydroxy et/ou amino, et M⁺ représente notamment un cation alcalin ou ammonium .

8. Ensemble de sondes selon l'une quelconques des revendications 4 à 6, **caractérisé par le fait que** ledit ligand comprend un groupement de formule générale (II) 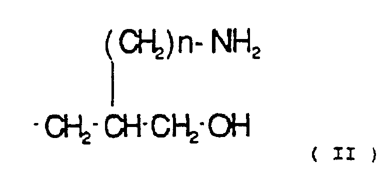 dans laquelle n est un nombre entier pouvant varier de 1 à 4

9. Ensemble de sondes selon l'une quelconques des revendications 4 à 6, **caractérisé par le fait que** ledit ligand comprend un peptide ou une protéine.

10. Ensemble de sondes selon la revendications 9, **caractérisé par le fait que** ladite protéine est une albumine

11. Ensemble de sondes selon la revendications 10, **caractérisé par le fait que** ladite albumine est une albumine de sérum de bovin.

12. Ensemble de sondes selon l'une quelconques des revendications précédentes, **caractérisé par le fait que** lesdites sondes sont fixées par adsorption passive sur ledit support solide.

13. Procédé pour déterminer le typage HLA DR d'un individu à partir d'un échantillon de l'individu selon les techniques usuelles de typage par oligonucléotides **caractérisé par le fait qu'**on utilise comme sondes de capture ou de détection au moins une partie des sondes de l'ensemble de sondes défini dans l'une quelconque des revendications précédentes, ladite partie de l'ensemble de sondes contenant au moins une sonde telle que définie représentées dans la revendication 1, partie (a), ou sa séquence complémentaire.

14. Procédé selon la revendication 13 **caractérisé par le fait qu'**il comprend les étapes consistant
- à faire incuber selon les méthodes connues dans des conditions prédéterminées telles que l'hybridation avec chaque sonde ne se produit que si la cible contient une séquence parfaitement complémentaire de celle de ladite sonde, et
- à déterminer selon les techniques de détection usuelles l'hybridation ou l'absence d'hybridation avec chacune des sondes utilisées.

15. Procédé selon les revendications 13 et 14. **caractérisé par le fait que** l'on utilise lesdites sondes comme sondes de capture.

16. Procédé selon la revendication 15, **caractérisé par le fait qu'**il comprend les étapes consistant à
- immobiliser chaque sonde de capture sur un support solide,
- mettre en contact chaque sonde de capture immobilisée avec un milieu liquide contenant au moins un fragment d'acide nucléique cible, dans des conditions prédéterminées permettant l'hybridation si la séquence complémentaire de celle de la sonde est présente dans la cible, et
- détecter la présence d'hybrides éventuellement formés.

17. Procédé selon la revendication 16, **caractérisé par le fait que** ladite sonde de capture est telle que définie dans l'une quelconque des revendications 4 à 12.

18. Procédé selon l'une quelconque des revendications 13 à 17. **caractérisé par le fait que** le fragment d'acides nucléiques cible est un fragment d'ADN ou d'ARN

19. Procédé selon l'une des revendications 14 à 16 **caractérisé par le fait que** la détection des hybrides formés est effectuée à l'aide d'au moins l'une des sondes de détection suivantes :

CCGGGCGGTGACGGAGCTGGGGC ; CCGGGCGGTGACIGAGCTGGGGC ;
GAACAGCCAGAAGGAC ; CCGGGCGGTGACTGAGCTGGGGC.

20. Sonde nucléotidique choisie parmi celles ayant les séquences suivantes ou leurs complémentaires: 
(G) CTGGAAAGATGCA (T)

CAGCAGGATAAGTATG (A)

(G) TGGACAACTACT (G)

GATACTTCTATCACC (AA)

(G) CCTGATGAGGAGTA (C)
(G) GGCCCTGGTGGA (CA)

(T) GCGGTATCTGCACA

GGAGGAGGTTAAG (TT)

(C) TGGAAGACGAGC (G)

(T) GGAAGACAAGCG (G)

(T) GCGGAGCACTGG (A)

(A) CCAGIAGGAGAACGT (G)

(A) CCAGGAGGAGAACGT (G)

(A) CTCTACGGGTGAGT (G)

(G) ACACCTATTGCAGA (C)

(G) GAGCTGCGTAAG (T)

TTCCTGGAGAGACAC

(G) GGAGAGATACTTC (C)

(G) AGGAGGACTTGCGC (T)

(T) ACGGGGCTGTGGA (G)

AACCAGIAGGAGAACGT

CCGGGCGGTGACGGAGCTGGGGC

CCGGGCGGTGACIGAGCTGGGGC

GAACAGCCAGAAGGAC

CCGGGCGGTGACTGAGCTGGGGC
 lesdites sondes pouvant être sous forme marquée, ou couplées de façon covalente à un ligand facilitant leur fixation sur un support solide, ou fixées sur un support solide.

## Patentansprüche

1. Kombination von Nucleotidsonden, die eine Bestimmung der Typen von HLA-DR und/oder assoziierten Subtypen nach üblichen Verfahrensweisen der Typisierung nach Oligonucleotiden erlauben, wobei die Kombination von Sonden wenigstens die folgenden Nucleotidsonden oder dazu komplementäre Sonden umfasst (der unterstrichene Teil entspricht der optimalen Sequenz, und der Buchstabe I steht für Inosin):
(a) 
(G)CTGGAAAGATGCA(T), CAGCAGGATAAGTATG (A), (G)TGGACAACTACT (G),
GATACTTCTATCACC(AA), (G)CCTGATGAGGAGTA(C),
(G)GGCCCTGGTGGA(CA), (T)GCGGTATCTGCACA,
GGAGGAGGTTAAG(TT), (T)GGAAGACAAGCG(G), (T)GCGGAGCACTGG(A),
(A)CCAGIAGGAGAACGT(G), (A)CCAGGAGGAGAACGT(G),
(A)CTCTACGGGTGAGT(G), (G)ACACCTATTGCAGA(C);
(b) (C)TGGAAGACGAGC(G); und
(c) (T)GGCAGGGTAAGTATAAG, wobei die Sonden in markierter Form vorliegen können oder kovalent an einen Liganden gebunden vorliegen können, der ihre Fixierung auf einem festen Träger erleichtert, oder auf einem festen Träger fixiert vorliegen können.

2. Kombination von Sonden nach Anspruch 1, umfassend zusätzlich die folgenden Sonden (der unterstrichene Teil entspricht der optimalen Sequenz): (G)GAGCTGCGTAAG(T), TTCCTGGAGAGACAC, (G)GGAGAGATACTTC(C), und/oder die folgenden Sonden:

(G)AGGAGGACTTGCGC(T), (T)ACGGGCTGTGGA(G).

3. Kombination von Sonden nach Anspruch 1 oder Anspruch 2, **dadurch gekennzeichnet, dass** sie die folgende Sonde enthält:

AACCAGIAGGAGAACGT.

4. Kombination von Sonden nach einem der vorhergehenden Ansprüche, **dadurch gekennzeichnet, dass** die Sonden kovalent an einen Liganden gebunden sind, insbesondere an einen Liganden, der wenigstens eine polare funktionelle Gruppe umfasst.

5. Kombination von Sonden nach Anspruch 4, **dadurch gekennzeichnet, dass** die polare funktionelle Gruppe eine Aminogruppe ist.

6. Kombination von Sonden nach einem der Ansprüche 4 bis 5, **dadurch gekennzeichnet, dass** der Ligand einen hydrophoben Teil umfasst.

7. Kombination von Sonden nach einem der Ansprüche 4 bis 6, **dadurch gekennzeichnet, dass** der Ligand eine Gruppe der allgemeinen Formel (I) umfasst, 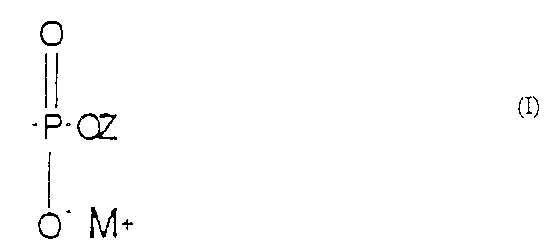 worin Z für einen Alkyl- oder Alkenyl-Rest mit 2 bis 12 Kohlenstoffatomen steht, der geradkettig oder verzweigt ist und unsubstituiert oder mit einer oder mehreren Hydroxy- und/oder Aminogruppen substituiert ist, und M⁺ insbesondere für ein Alkalimetall- oder Ammonium-Kation steht.

8. Kombination von Sonden nach einem der Ansprüche 4 bis 6, **dadurch gekennzeichnet, dass** der Ligand eine Gruppe der allgemeinen Formel (II) umfasst, 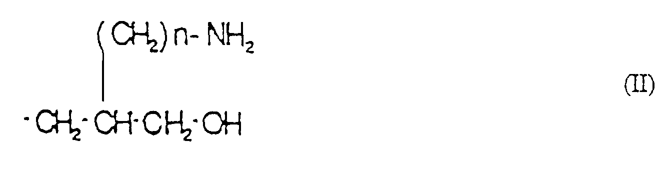 worin n für eine ganze Zahl steht, die von 1 bis 4 schwanken kann.

9. Kombination von Sonden nach einem der Ansprüche 4 bis 6, **dadurch gekennzeichnet, dass** der Ligand ein Peptid oder ein Protein umfassen kann.

10. Kombination von Sonden nach Anspruch 9, **dadurch gekennzeichnet, dass** das Protein ein Albumin ist.

11. Kombination von Sonden nach Anspruch 10, **dadurch gekennzeichnet, dass** das Albumin Rinderserum-Albumin ist.

12. Kombination von Sonden nach einem der vorhergehenden Ansprüche, **dadurch gekennzeichnet, dass** die Sonden durch passive Adsorption auf dem festen Träger fixiert sind.

13. Verfahren zum Bestimmen der Typen von HLA-DR eines Individuums anhand einer Probe des Individuums nach üblichen Verfahren der Typisierung nach Oligonucleotiden, **dadurch gekennzeichnet, dass** man als Fängersonden oder Nachweissonden wenigstens einen Teil der Sonden der Kombination von Sonden verwendet, wie sie in einem der vorhergehenden Ansprüche definiert sind, wobei der Teil der Kombination von Sonden wenigstens eine Sonde enthält, wie sie unter (a) in Anspruch 1 wiedergegeben definiert ist, oder eine dazu komplementäre Sequenz.

14. Verfahren nach Anspruch 13, **dadurch gekennzeichnet, dass** es die Schritte umfasst, die bestehen aus
- Inkubieren nach bekannten Verfahrensweisen unter vorbestimmten Bedingungen wie Hybridisierung mit jeder Sonde, die nur dann abläuft wenn die Zielverbindung eine Sequenz enthält, die perfekt zu derjenigen der Sonde komplementär ist; und
- Bestimmen der Hybridisierung oder fehlenden Hybridisierung mit einer der verwendeten Sonden mit üblichen Nachweistechniken.

15. Verfahren nach den Ansprüchen 13 und 14, **dadurch gekennzeichnet, dass** man die Sonden als Fängersonden verwendet.

16. Verfahren nach Anspruch 15, **dadurch gekennzeichnet, dass** es die Schritte umfasst, die bestehen aus
- Immobilisieren jeder Fängersonde auf einem festen Träger;
- In-Kontakt-Bringen jeder immobilisierten Fängersonde mit einem flüssigen Medium, das wenigstens ein Fragment einer Ziel-Nucleinsäure enthält, unter vorbestimmten Bedingungen, die die Hybridisierung erlauben, wenn die zu der Sequenz der Sonde komplementäre Sequenz in der Zielverbindung zugegen ist; und
- Nachweisen des Vorhandenseins eventuell gebildeter Hybride.

17. Verfahren nach Anspruch 16, **dadurch gekennzeichnet, dass** die Fängersonde die Sonde ist, die in einem der Ansprüche 4 bis 12 definiert ist.

18. Verfahren nach einem der Ansprüche 13 bis 17, **dadurch gekennzeichnet, dass** das Ziel-Nucleinsäurefragment ein Fragment einer DNS oder einer RNS ist.

19. Verfahren nach einem der Ansprüche 14 bis 16, **dadurch gekennzeichnet, dass** der Nachweis der gebildeten Hybride mittels wenigstens einer der folgenden Nachweissonden bewirkt wird:

CCGGGCGGTGACGGAGCTGGGGC; CCGGGCGGTGACIGAGCTGGGGC;
GAACAGCCAGAAGGAC; CCGGGCGGTGACTGAGCTGGGGC.

20. Nucleotidsonde, gewählt unter den Sonden, die die folgenden Sequenzen haben, oder dazu komplementäre Sequenzen: 
(G) CTGGAAAGATGCA (T)

CAGCAGGATAAGTATG (A)

(G) TGGACAACTACT (G)

GATACTTCTATCACC (AA)

(G) CCTGATGAGGAGTA (C)

(G) GGCCCTGGTGGA (CA)

(T) GCGGTATCTGCACA

GGAGGAGGTTAAG (TT)

(C) TGGAAGACGAGC (G)

(T) GGAAGACAAGCG (G)

(T) GCGGAGCACTGG (A)

(A) CCAGIAGGAGAACGT (G)

(A) CCAGGAGGAGAACGT (G)
 
(A) CTCTACGGGTGAGT (G)
(G) ACACCTATTGCAGA (C)
(G) GAGCTGCGTAAG (T)
TTCCTGGAGAGACAC
(G) GGAGAGATACTTC (C)
(G) AGGAGGACTTGCGC (T)
(T) ACGGGGCTGTGGA (G)
AACCAGIAGGAGAACGT
CCGGGCGGTGACGGAGCTGGGGC
CCGGGCGGTGACIGAGCTGGGGC
GAACAGCCAGAAGGAC
CCGGGCGGTGACTGAGCTGGGGC
 wobei die Sonden in markierter Form vorliegen können oder kovalent an einen Liganden gebunden vorliegen können, der ihre Fixierung auf einem festen Träger erleichtert, oder auf einem festen Träger fixiert vorliegen können.

## Claims

1. Set of nucleotide probes enabling the HLA-DR types and/or associated subtypes to be determined according to the standard techniques of typing with oligonucleotides, the said set of probes comprising at least the following nucleotide probes or their complementary sequences (the underlined portion corresponding to the optimum sequence, and the letter I representing inosine):
(a): (G)CTGGAAAGATGCA(T),  CAGCAGGATAAGTATG(A), 
(G)TGGACAACTACT(G),                GATACTTCTATCACC(AA),
(G)CCTGATGAGGAGTA(C),                (G)GGCCCTGGTGGA(CA),
(T)GCGGTATCTGCACA, GGAGGAGGTTAAG (TT), (T)GGAAGAC-
AAGCG(G),      (T)GCGGAGCACTGG(A),      (A)CCAGIAGGAG-
AACGT(G),                          (A)CCAGGAGGAGAACGT(G),
(A)CTCTACGGGTGAGT(G), (G)ACACCTATTGCAGA(C)
(b): (C)TGGAAGACGAGC(G), and
(c) (T) GGCAGGGTAAGTATAAG, the bases in brackets being optional,
it being possible for the said probes to be in labelled form, or coupled covalently to a ligand facilitating their binding to a solid support, or bound to a solid support.

2. Set of probes according to Claim 1, comprising, in addition, the following probes (the underlined portion corresponding to the optimum sequence) 
(G)GAGCTGCGTAAG(T),                    TTCCTGGAGAGACAC,
(G)GGAGAGATACTTC(C)
 and/or the following probes

(G)AGGAGGACTTGCGC(T), (T)ACGGGGCTGTGGA(G).

3. Set of probes according to Claim 1 or 2, **characterized in that** it contains the following probe:

AACCAGIAGGAGAACGT.

4. Set of probes according to any one of the preceding claims, **characterized in that** the said probes are coupled covalently with a ligand, especially with a ligand comprising at least one polar functional group.

5. Set of probes according to Claim 4, **characterized in that** the said polar functional group is an amino group.

6. Set of probes according to one of Claims 4 to 5, **characterized in that** the said ligand comprises a hydrophobic portion.

7. Set of probes according to any one of Claims 4 to 6, **characterized in that** the said ligand comprises a group of general formula (I) 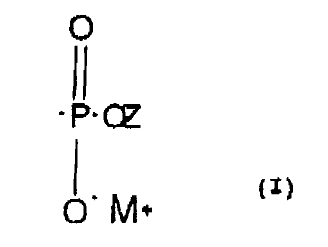 in which Z represents a linear or branched alkyl or alkenyl radical having 2 to 12 carbon atoms, unsubstituted or substituted with one or more hydroxyl and/or amino groups, and M⁺ represents, in particular, an alkali metal or ammonium cation.

8. Set of probes according to any one of Claims 4 to 6, **characterized in that** the said ligand comprises a group of general formula (II) 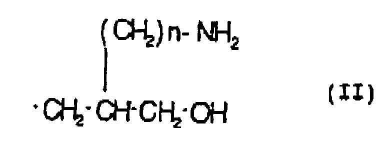 in which n is an integer which can vary from 1 to 4.

9. Set of probes according to any one of Claims 4 to 6, **characterized in that** the said ligand comprises a peptide or a protein.

10. Set of probes according to Claim 9, **characterized in that** the said protein is an albumin

11. Set of probes according to Claim 10, **characterized in that** the said albumin is a bovine serum albumin.

12. Set of probes according to any one of the preceding claims, **characterized in that** the said probes are bound by passive adsorption on the said solid support.

13. Method for determining an individual's HLA-DR typing from a sample from the individual, according to the standard techniques of typing with oligonucleotides, **characterized in that** at least a portion of the probes of the set of probes defined in any one of the preceding claims is used as capture or detection probes, the said portion of the set of probes containing at least one probe as defined in Claim 1, part (a), or its complementary sequence.

14. Method according to Claim 13, **characterized in that** it comprises the steps consisting in:
- incubating according to known methods under predetermined conditions such that hybridization with each probe takes place only if the target contains a sequence fully complementary to that of the said probe, and
- determining, according to standard detection techniques, the hybridization or lack of hybridization with each of the probes used.

15. Method according to Claims 13 and 14, **characterized in that** the said probes are used as capture probes.

16. Method according to Claim 15, **characterized in that** it comprises the steps consisting in:
- immobilising each capture probe on a solid support,
- bringing each immobilised capture probe into contact with a liquid medium containing at least one target nucleic acid fragment, under predetermined conditions permitting hybridization if the sequence complementary to that of the probe is present in the target, and
- detecting the presence of any hybrids which may be formed.

17. Method according to Claim 16, **characterized in that** the said capture probe is as defined in any one of Claims 4 to 12.

18. Method according to any one of Claims 13 to 17, **characterized in that** the target nucleic acid fragment is a DNA or RNA fragment.

19. Method according to one of Claims 14 to 16, **characterized in that** the detection of the hybrids formed is performed using at least one of the following detection probes:

CCGGGCGGTGACGGAGCTGGGGC;    CCGGGCGGTGACIGAGCTGGGGC;
GAACAGCCAGAAGGAC; CCGGGCGGTGACTGAGCTGGGGC.

20. Nucleotide probe chosen from those having the following sequences, or their complementary sequences: 
(G)CTGGAAAGATGCA(T)
CAGCAGGATAAGTATG(A)
(G)TGGACAACTACT(G)
GATACTTCTATCACC(AA)
(G)CCTGATGAGGAGTA(C)
(G)GGCCCTGGTGGA(CA)
(T)GCGGTATCTGCACA
GGAGGAGGTTAAG(TT)
(C)TGGAAGACGAGC(G)
(T)GGAAGACAAGCG(G)
(T)GCGGAGCACTGG(A)
(A)CCAGIAGGAGAACGT(G)
(A)CCAGGAGGAGAACGT(G)
(A)CTCTACGGGTGAGT(G)
(G)ACACCTATTGCAGA(C)
(G)GAGCTGCGTAAG(T)
TTCCTGGAGAGACAC
(G)GGAGAGATACTTC(C)
(G)AGGAGGACTTGCGC(T)
(T)ACGGGGCTGTGGA(G)
AACCAGIAGGAGAACGT
CCGGGCGGTGACGGAGCTGGGGC
CCGGGCGGTGACIGAGCTGGGGC
GAACAGCCAGAAGGAC
CCGGGCGGTGACTGAGCTGGGGC
 it being possible for the said probes to be in labelled form, or coupled covalently to a ligand facilitating their binding to a solid support, or bound to a solid support.
